# EUROPEAN PATENT APPLICATION

(11) **EP 2 270 168 A1**
(43) Date of publication of application: **05.01.2011**
(21) Application number: 09715272.2
(22) Date of filing: 23.02.2009
(51) Int. Cl.: C12N 15/29, A01H 5/00, C07K 14/415, C12N 5/10

(54) **MUTANT GENE CAPABLE OF CONTROLLING THE OIL-AND-FAT CONTENT IN SEED, AND METHOD FOR CONTROLLING THE OIL-AND-FAT CONTENT IN SEED**

(30) Priority: 28.02.2008 JP 2008048612
(71) Applicant: Toyota Jidosha Kabushiki Kaisha, Toyota-shi, Aichi 471-8571 (JP)
(72) Inventor: OHTO, Chikara, Toyota-shi Aichi 471-8571 (JP); MITSUKAWA, Norihiro, Toyota-shi Aichi 471-8571 (JP); HAYASHI, Makoto, Okazaki-shi Aichi 444-8585 (JP)
(74) Representative: O'Brien, Simon Warwick
(86) International application number: PCT/JP2009/053784
(87) International publication number: WO 2009/107822

(57) **Abstract**

This invention provides a mutant gene having the function of regulating the oil-and-fat content in seeds and a method for regulating the oil-and-fat content in seeds. The mutant gene encodes a mutant protein having a consensus sequence resulting from substitution of the 12th amino acid residue from the N-terminus of the amino acid sequence as shown in SEQ ID NO: 1 with another amino acid and having the function of regulating the oil-and-fat content in seeds.

SEQUENCE LISTING

<110>  TOYOTA JIDOSHA KABUSHIKI KAISHA

<120>  A mutant gene capable of regulating an amount of oils in seeds

<130>  PH-3780-PCT

<150>  JP 2008-048612
<151>  2008-02-28

<160>  25

<170>  PatentIn version 3.4

<210>  1
<211>  80
<212>  PRT
<213>  Artificial

<220>
<223>  A consensus sequence including a DAGAT domain

<220>
<221>  misc_feature
<222>  (1)..(1)
<223>  Xaa of position 1 of SEQ ID No:1 represents D or E

<220>
<221>  misc_feature
<222>  (2)..(6)
<223>  Each of Xaas of position 2 to 6 of SEQ ID No:1 represents an arbitrary
amino acid

<220>
<221>  misc_feature
<222>  (8)..(11)
<223>  Each of Xaas of position 8 to 11 of SEQ ID No:1 represents an arbitrary
amino acid

<220>

<221>  misc_feature
<222>  (12)..(12)
<223>  Xaa of position 12 of SEQ ID No:1 represents T or S

<220>
<221>  misc_feature
<222>  (13)..(16)
<223>  Each of Xaas of position 13 to 16 of SEQ ID No:1 represents an arbitrary
amino acid

<220>
<221>  misc_feature
<222>  (17)..(17)
<223>  Xaa of position 17 of SEQ ID No:1 represents Y or F .

<220>
<221>  misc_feature
<222>  (18)..(18)
<223>  Xaa of position 18 of SEQ ID No:1 represents R, K, N, or Q

<220>
<221>  misc_feature
<222>  (20)..(20)
<223>  Xaa of position 20 of SEQ ID No:1 represents F or L

<220>
<221>  misc_feature
<222>  (21)..(24)
<223>  Each of Xaas of position 21 to 24 of SEQ ID No:1 represents an arbitrary
amino acid

<220>
<221>  misc_feature
<222>  (25)..(25)
<223>  Xaa of position 25 of SEQ ID No:1 represents F or H

<220>
<221>  misc_feature
<222>  (27)..(27)
<223>  Xaa of position 27 of SEQ ID No:1 represents an arbitrary amino acid

<220>

<221> misc_feature
<222> (29)..(29)
<223> Xaa of position 29 of SEQ ID No:1 represents Y or F

<220>
<221> misc_feature
<222> (33)..(33)
<223> Xaa of position 33 of SEQ ID No:1 represents an arbitrary amino acid

<220>
<221> misc_feature
<222> (36)..(36)
<223> Xaa of position 36 of SEQ ID No:1 represents A or S

<220>
<221> misc_feature
<222> (39)..(39)
<223> Xaa of position 39 of SEQ ID No:1 represents an arbitrary amino acid

<220>
<221> misc_feature
<222> (40)..(40)
<223> Xaa of position 40 of SEQ ID No:1 represents K or R

<220>
<221> misc_feature
<222> (41)..(41)
<223> Xaa of position 41 of SEQ ID No:1 represents G or D

<220>
<221> misc_feature
<222> (43)..(43)
<223> Xaa of position 43 of SEQ ID No:1 represents an arbitrary amino acid

<220>
<221> misc_feature
<222> (44)..(44)
<223> Xaa of position 44 of SEQ ID No:1 represents Y or H

<220>
<221> misc_feature
<222> (45)..(45)

<223>  Xaa of position 45 of SEQ ID No:1 represents K, R or Q

<220>
<221>  misc_feature
<222>  (47)..(47)
<223>  Xaa of position 47 of SEQ ID No:1 represents F or I

<220>
<221>  misc_feature
<222>  (50)..(50)
<223>  Xaa of position 50 of SEQ ID No:1 represents D or E

<220>
<221>  misc_feature
<222>  (51)..(51)
<223>  Xaa of position 51 of SEQ ID No:1 represents Q, H or R

<220>
<221>  misc_feature
<222>  (52)..(52)
<223>  Xaa of position 52 of SEQ ID No:1 represents an arbitrary amino acid

<220>
<221>  misc_feature
<222>  (57)..(57)
<223>  Xaa of position 57 of SEQ ID No:1 represents an arbitrary amino acid

<220>
<221>  misc_feature
<222>  (59)..(59)
<223>  Xaa of position 59 of SEQ ID No:1 represents A or S

<220>
<221>  misc_feature
<222>  (60)..(60)
<223>  Xaa of position 60 of SEQ ID No:1 represents R, K or Q

<220>
<221>  misc_feature
<222>  (62)..(62)
<223>  Xaa of position 62 of SEQ ID No:1 represents G or N

<220>
<221> misc_feature
<222> (63)..(63)
<223> Xaa of position 63 of SEQ ID No:1 represents A, V or T

<220>
<221> misc_feature
<222> (64)..(64)
<223> Xaa of position 64 of SEQ ID No:1 represents T or K

<220>
<221> misc_feature
<222> (65)..(65)
<223> Xaa of position 65 of SEQ ID No:1 represents I or V

<220>
<221> misc_feature
<222> (66)..(66)
<223> Xaa of position 66 of SEQ ID No:1 represents I or V

<220>
<221> misc_feature
<222> (70)..(70)
<223> Xaa of position 70 of SEQ ID No:1 represents an arbitrary amino acid

<220>
<221> misc_feature
<222> (76)..(76)
<223> Xaa of position 76 of SEQ ID No:1 represents V, F, I, L or M

<220>
<221> misc_feature
<222> (77)..(77)
<223> Xaa of position 77 of SEQ ID No:1 represents an arbitrary amino acid

<220>
<221> misc_feature
<222> (78)..(78)
<223> Xaa of position 78 of SEQ ID No:1 represents E, D or H

<220>
<221> misc_feature

<222>  (79)..(79)
<223>  Xaa of position 79 of SEQ ID No:1 represents L, V, M or I

<220>
<221>  misc_feature
<222>  (80)..(80)
<223>  Xaa of position 80 of SEQ ID No:1 represents an arbitrary amino acid

<400>  1

Xaa Xaa Xaa Xaa Xaa Xaa Gly Xaa Xaa Xaa Xaa Xaa Xaa Xaa Xaa Xaa
1               5                   10                  15

Xaa Xaa Leu Xaa Xaa Xaa Xaa Xaa Xaa Val Xaa Leu Xaa Pro Gly Gly
            20                  25                  30

Xaa Arg Glu Xaa Leu His Xaa Xaa Xaa Glu Xaa Xaa Xaa Leu Xaa Trp
        35                  40                  45

Pro Xaa Xaa Xaa Glu Phe Val Arg Xaa Ala Xaa Xaa Phe Xaa Xaa Xaa
    50                  55                  60

Xaa Xaa Pro Phe Gly Xaa Val Gly Glu Asp Asp Xaa Xaa Xaa Xaa Xaa
65                  70                  75                  80

<210>  2
<211>  90
<212>  PRT
<213>  Artificial

<220>
<223>  A consensus sequence including a DAGAT domain

<220>
<221>  misc_feature
<222>  (1)..(1)

<223>  Xaa of position 1 of SEQ ID No:2 represents D or E

<220>
<221>  misc_feature
<222>  (2)..(6)
<223>  Each of Xaas of position 2 to 6 of SEQ ID No:2 represents an arbitrary
amino acid

<220>
<221>  misc_feature
<222>  (8)..(11)
<223>  Each of Xaas of position 8 to 11 of SEQ ID No:2 represents an arbitrary
amino acid

<220>
<221>  misc_feature
<222>  (12)..(12)
<223>  Xaa of position 12 of SEQ ID No:2 represents T or S

<220>
<221>  misc_feature
<222>  (13)..(16)
<223>  Each of Xaas of position 13 to 16 of SEQ ID No:2 represents an arbitrary
amino acid

<220>
<221>  misc_feature
<222>  (17)..(17)
<223>  Xaa of position 17 of SEQ ID No:2 represents Y or F

<220>
<221>  misc_feature
<222>  (18)..(18)
<223>  Xaa of position 18 of SEQ ID No:2 represents R, K, N, or Q

<220>
<221>  misc_feature
<222>  (20)..(20)
<223>  Xaa of position 20 of SEQ ID No:2 represents F or L

<220>
<221>  misc_feature

<222>  (21)..(24)
<223>  Each of Xaas of position 21 to 24 of SEQ ID No:2 represents an arbitrary
amino acid

<220>
<221>  misc_feature
<222>  (25)..(25)
<223>  Xaa of position 25 of SEQ ID No:2 represents F or H

<220>
<221>  misc_feature
<222>  (27)..(27)
<223>  Xaa of position 27 of SEQ ID No:2 represents an arbitrary amino acid

<220>
<221>  misc_feature
<222>  (29)..(29)
<223>  Xaa of position 29 of SEQ ID No:2 represents Y or F

<220>
<221>  misc_feature
<222>  (33)..(33)
<223>  Xaa of position 33 of SEQ ID No:2 represents an arbitrary amino acid

<220>
<221>  misc_feature
<222>  (36)..(36)
<223>  Xaa of position 36 of SEQ ID No:2 represents A or S

<220>
<221>  misc_feature
<222>  (39)..(39)
<223>  Xaa of position 39 of SEQ ID No:2 represents an arbitrary amino acid

<220>
<221>  misc_feature
<222>  (40)..(40)
<223>  Xaa of position 40 of SEQ ID No:2 represents K or R

<220>
<221>  misc_feature
<222>  (41)..(41)

<223> Xaa of position 41 of SEQ ID No:2 represents G or D

<220>
<221> misc_feature
<222> (43)..(43)
<223> Xaa of position 43 of SEQ ID No:2 represents an arbitrary amino acid

<220>
<221> misc_feature
<222> (44)..(44)
<223> Xaa of position 44 of SEQ ID No:2 represents Y or H

<220>
<221> misc_feature
<222> (45)..(45)
<223> Xaa of position 45 of SEQ ID No:2 represents K, R or Q

<220>
<221> misc_feature
<222> (47)..(47)
<223> Xaa of position 47 of SEQ ID No:2 represents F or I

<220>
<221> misc_feature
<222> (50)..(50)
<223> Xaa of position 50 of SEQ ID No:2 represents D or E

<220>
<221> misc_feature
<222> (51)..(51)
<223> Xaa of position 51 of SEQ ID No:2 represents Q, H or R

<220>
<221> misc_feature
<222> (52)..(52)
<223> Xaa of position 52 of SEQ ID No:2 represents an arbitrary amino acid

<220>
<221> misc_feature
<222> (57)..(57)
<223> Xaa of position 57 of SEQ ID No:2 represents an arbitrary amino acid

<220>
<221> misc_feature
<222> (59)..(59)
<223> Xaa of position 59 of SEQ ID No:2 represents A or S

<220>
<221> misc_feature
<222> (60)..(60)
<223> Xaa of position 60 of SEQ ID No:2 represents R, K or Q

<220>
<221> misc_feature
<222> (62)..(62)
<223> Xaa of position 62 of SEQ ID No:2 represents G or N

<220>
<221> misc_feature
<222> (63)..(63)
<223> Xaa of position 63 of SEQ ID No:2 represents A, V or T

<220>
<221> misc_feature
<222> (64)..(64)
<223> Xaa of position 64 of SEQ ID No:2 represents T or K

<220>
<221> misc_feature
<222> (65)..(65)
<223> Xaa of position 65 of SEQ ID No:2 represents I or V

<220>
<221> misc_feature
<222> (66)..(66)
<223> Xaa of position 66 of SEQ ID No:2 represents I or V

<220>
<221> misc_feature
<222> (70)..(70)
<223> Xaa of position 70 of SEQ ID No:2 represents an arbitrary amino acid

<220>
<221> misc_feature

<222>  (76)..(76)
<223>  Xaa of position 76 of SEQ ID No:2 represents V, F, I, L or M

<220>
<221>  misc_feature
<222>  (77)..(77)
<223>  Xaa of position 77 of SEQ ID No:2 represents an arbitrary amino acid

<220>
<221>  misc_feature
<222>  (78)..(78)
<223>  Xaa of position 78 of SEQ ID No:2 represents E, D or H

<220>
<221>  misc_feature
<222>  (79)..(79)
<223>  Xaa of position 79 of SEQ ID No:2 represents L, V, M or I

<220>
<221>  misc_feature
<222>  (80)..(83)
<223>  Each of Xaas of position 80 to 83 of SEQ ID No:2 represents an arbitrary
amino acid

<220>
<221>  misc_feature
<222>  (84)..(84)
<223>  Xaa of position 84 of SEQ ID No:2 represents N, H, E or D

<220>
<221>  misc_feature
<222>  (85)..(85)
<223>  Xaa of position 85 of SEQ ID No:2 represents D or E

<220>
<221>  misc_feature
<222>  (86)..(87)
<223>  Each of Xaas of position 86 to 87 of SEQ ID No:2 represents an arbitrary
amino acid

<220>
<221>  misc_feature

<222>  (88)..(88)
<223>  Xaa of position 88 of SEQ ID No:2 represents N or K

<220>
<221>  misc_feature
<222>  (89)..(89)
<223>  Xaa of position 89 of SEQ ID No:2 represents an arbitrary amino acid

<400>  2

Xaa Xaa Xaa Xaa Xaa Xaa Gly Xaa Xaa Xaa Xaa Xaa Xaa Xaa Xaa Xaa
1               5                   10                  15

Xaa Xaa Leu Xaa Xaa Xaa Xaa Xaa Xaa Val Xaa Leu Xaa Pro Gly Gly
            20                  25                  30

Xaa Arg Glu Xaa Leu His Xaa Xaa Xaa Glu Xaa Xaa Xaa Leu Xaa Trp
        35                  40                  45

Pro Xaa Xaa Xaa Glu Phe Val Arg Xaa Ala Xaa Xaa Phe Xaa Xaa Xaa
    50                  55                  60

Xaa Xaa Pro Phe Gly Xaa Val Gly Glu Asp Asp Xaa Xaa Xaa Xaa Xaa
65                  70                  75                  80

Xaa Xaa Xaa Xaa Xaa Xaa Xaa Xaa Xaa Pro
                85                  90

<210>  3
<211>  2115
<212>  DNA
<213>  Arabidopsis thaliana

<220>
<221>  CDS

<222>  (1)..(2115)

<400>  3
atg gca aca tgt tct tca agc tta ttg gtg tta cca aat ttg aga ttg      48
Met Ala Thr Cys Ser Ser Ser Leu Leu Val Leu Pro Asn Leu Arg Leu
1               5                   10                  15

agt tct aac caa aga cgc aat ttc aag gta aga gct caa atc tct ggt      96
Ser Ser Asn Gln Arg Arg Asn Phe Lys Val Arg Ala Gln Ile Ser Gly
            20                  25                  30

gaa aac aag aag gcg aca tca ttg gaa cca gtg aac aac aac ggt tcg      144
Glu Asn Lys Lys Ala Thr Ser Leu Glu Pro Val Asn Asn Asn Gly Ser
        35                  40                  45

gtc tct ttg tct act act gtt caa aac caa aaa ggg gca aat gaa gtg      192
Val Ser Leu Ser Thr Thr Val Gln Asn Gln Lys Gly Ala Asn Glu Val
    50                  55                  60

aat ggg aaa ggg aaa tcc aag aga aag ata gtg agt gat gaa att gag      240
Asn Gly Lys Gly Lys Ser Lys Arg Lys Ile Val Ser Asp Glu Ile Glu
65                  70                  75                  80

ttg tta tgg gat gat ggg tat ggc tcc aaa tct gtt aaa gat tac ttt      288
Leu Leu Trp Asp Asp Gly Tyr Gly Ser Lys Ser Val Lys Asp Tyr Phe
                85                  90                  95

gct gcg gct aaa gag att ttg aag gct gat ggt ggt cct cca cgg tgg      336
Ala Ala Ala Lys Glu Ile Leu Lys Ala Asp Gly Gly Pro Pro Arg Trp
            100                 105                 110

ttt agt ccc gtt gat tgt ggt cga cct gtt gaa gat gct cct acg tta      384
Phe Ser Pro Val Asp Cys Gly Arg Pro Val Glu Asp Ala Pro Thr Leu
        115                 120                 125

ctc ttc tta cct ggg atg gat ggc aca ggg atg ggc ctt gtt cca cat      432
Leu Phe Leu Pro Gly Met Asp Gly Thr Gly Met Gly Leu Val Pro His
    130                 135                 140

cac aaa gct ctt ggc aag gct ttt cat gtc tcg tgc ctg cat att cct      480
His Lys Ala Leu Gly Lys Ala Phe His Val Ser Cys Leu His Ile Pro
145                 150                 155                 160

gta ctt gat cga act cca ttt gaa ggg ctt ctg aaa gta gtc gaa gat 528
Val Leu Asp Arg Thr Pro Phe Glu Gly Leu Leu Lys Val Val Glu Asp
                165                 170                 175

gtt ttg agg cag gag cag gct aca cgt cct aat aag ccg ata tat ctc 576
Val Leu Arg Gln Glu Gln Ala Thr Arg Pro Asn Lys Pro Ile Tyr Leu
            180                 185                 190

gtg ggc gat tcc ttt gga gga tgc ctg gct ctt gct gtt gct gct aga 624
Val Gly Asp Ser Phe Gly Gly Cys Leu Ala Leu Ala Val Ala Ala Arg
        195                 200                 205

aac cgt tcg tta gac ttg gtg ttg ata cta gtc aac cca gct aca tca 672
Asn Arg Ser Leu Asp Leu Val Leu Ile Leu Val Asn Pro Ala Thr Ser
    210                 215                 220

ttt gat agg tca ccg cta cag ccg ttg ctg cct ata tta gag atg gtg 720
Phe Asp Arg Ser Pro Leu Gln Pro Leu Leu Pro Ile Leu Glu Met Val
225                 230                 235                 240

cca gag gag ctt cat ttt act gtt cca tat gct cta agc ttt att atg 768
Pro Glu Glu Leu His Phe Thr Val Pro Tyr Ala Leu Ser Phe Ile Met
                245                 250                 255

ggt gat ccg ata aag atg gcg aca ctt ggt atc gac aac caa ctt cca 816
Gly Asp Pro Ile Lys Met Ala Thr Leu Gly Ile Asp Asn Gln Leu Pro
            260                 265                 270

aca gga gta aaa att gaa aag ttg aga caa agg ctt act aaa aca atg 864
Thr Gly Val Lys Ile Glu Lys Leu Arg Gln Arg Leu Thr Lys Thr Met
        275                 280                 285

ctc ccg ctt ctc tct gag ctc ggt gga att att cca cgg gaa acc tta 912
Leu Pro Leu Leu Ser Glu Leu Gly Gly Ile Ile Pro Arg Glu Thr Leu
    290                 295                 300

ctt tgg aag ctt aag tta ctg aga tct ggc tgt gct tat gcc aat tcc 960
Leu Trp Lys Leu Lys Leu Leu Arg Ser Gly Cys Ala Tyr Ala Asn Ser
305                 310                 315                 320

cgc atc cat gct gtt caa gct gaa gtc ttg gtc ctt gct agt gga aag 1008

Arg Ile His Ala Val Gln Ala Glu Val Leu Val Leu Ala Ser Gly Lys
                325                 330                 335

gac atg atg ctt cct agc caa gaa gaa gca aag cgg ctt cac ggg tta 1056
Asp Met Met Leu Pro Ser Gln Glu Glu Ala Lys Arg Leu His Gly Leu
            340                 345                 350

ctt aaa aac tgt tca gtt cga tgc ttc aag gac aat ggg cat acc ctt 1104
Leu Lys Asn Cys Ser Val Arg Cys Phe Lys Asp Asn Gly His Thr Leu
        355                 360                 365

tta ttg gaa gat agc atc agt ttg ctc aca gtt ata aaa ggt aca ggc 1152
Leu Leu Glu Asp Ser Ile Ser Leu Leu Thr Val Ile Lys Gly Thr Gly
    370                 375                 380

aag tac cgg cgg tcc tgg aga tat gat ttg gtt tct gat ttc ttg cct 1200
Lys Tyr Arg Arg Ser Trp Arg Tyr Asp Leu Val Ser Asp Phe Leu Pro
385                 390                 395                 400

cca agt aag gga gaa ctt gct tac gcc ctt gat gaa gtg ctt gga ttc 1248
Pro Ser Lys Gly Glu Leu Ala Tyr Ala Leu Asp Glu Val Leu Gly Phe
                405                 410                 415

ttg cga aat gct gtt ggt tct gtg ttt ttt tca act atg gaa gac ggg 1296
Leu Arg Asn Ala Val Gly Ser Val Phe Phe Ser Thr Met Glu Asp Gly
            420                 425                 430

aaa ata gtg aag gga ctt gct ggt gtt cca gat aaa ggt ccg gtg cta 1344
Lys Ile Val Lys Gly Leu Ala Gly Val Pro Asp Lys Gly Pro Val Leu
        435                 440                 445

ctt gtt ggt tac cac atg ttg atg ggt ctt gaa ctt ggt cca atg tcc 1392
Leu Val Gly Tyr His Met Leu Met Gly Leu Glu Leu Gly Pro Met Ser
    450                 455                 460

gag gcg ttt ata aaa gag aag aac att ctc ttt cgt gga atg gcc cat 1440
Glu Ala Phe Ile Lys Glu Lys Asn Ile Leu Phe Arg Gly Met Ala His
465                 470                 475                 480

ccg gtt ctg tat tct gat aat gat cca gct aag gcg ttt gat tat ggt 1488
Pro Val Leu Tyr Ser Asp Asn Asp Pro Ala Lys Ala Phe Asp Tyr Gly
                485                 490                 495

gac tgg att aaa gta ttt ggt gcc tat cct gtc acc gca acc aat cta 1536
Asp Trp Ile Lys Val Phe Gly Ala Tyr Pro Val Thr Ala Thr Asn Leu
            500                 505                 510

ttt aag ctg tta gat tct aaa tct cat gtc ctt ctc ttc cct ggt ggt 1584
Phe Lys Leu Leu Asp Ser Lys Ser His Val Leu Leu Phe Pro Gly Gly
        515                 520                 525

gca cga gag gct ctc cat aac cgg ggt gaa caa tac aaa cta att tgg 1632
Ala Arg Glu Ala Leu His Asn Arg Gly Glu Gln Tyr Lys Leu Ile Trp
    530                 535                 540

cct gaa cag caa gag ttt gta aga atg gca gca cgg ttt ggt gca aca 1680
Pro Glu Gln Gln Glu Phe Val Arg Met Ala Ala Arg Phe Gly Ala Thr
545                 550                 555                 560

ata gtg ccg ttt gga aca gtt ggt gaa gac gac ata gct gaa ttg gtt 1728
Ile Val Pro Phe Gly Thr Val Gly Glu Asp Asp Ile Ala Glu Leu Val
                565                 570                 575

ctt gat tac aac gac ctg atg aag atc cct att ctt aat gac tat atc 1776
Leu Asp Tyr Asn Asp Leu Met Lys Ile Pro Ile Leu Asn Asp Tyr Ile
            580                 585                 590

acg gaa gtt act cgc gat acc aaa caa ttt aaa ttg agg gaa gag tca 1824
Thr Glu Val Thr Arg Asp Thr Lys Gln Phe Lys Leu Arg Glu Glu Ser
        595                 600                 605

gag gga gag gtg gca aac cag cca ctc tac cta cca gga ctc ata ccg 1872
Glu Gly Glu Val Ala Asn Gln Pro Leu Tyr Leu Pro Gly Leu Ile Pro
    610                 615                 620

aaa gtc ccg ggt agg ttc tat tac ctg ttt ggg aaa ccg ata gag aca 1920
Lys Val Pro Gly Arg Phe Tyr Tyr Leu Phe Gly Lys Pro Ile Glu Thr
625                 630                 635                 640

aag gga aga cca gag ctt gta aaa gac aaa gaa gaa gca aat caa gtt 1968
Lys Gly Arg Pro Glu Leu Val Lys Asp Lys Glu Glu Ala Asn Gln Val
                645                 650                 655

tac ttg gag gta aag gcg gag gta gag aat agt ata gcc tac ctg ttg 2016

Tyr Leu Glu Val Lys Ala Glu Val Glu Asn Ser Ile Ala Tyr Leu Leu
            660                 665                 670

aag aaa cga gag gaa gat cca tac aga agt gtc tta gac aga tta aac 2064
Lys Lys Arg Glu Glu Asp Pro Tyr Arg Ser Val Leu Asp Arg Leu Asn
        675                 680                 685

tac agt ttg acg cac act acc gca act cat gtt cca tct ttc gag cca 2112
Tyr Ser Leu Thr His Thr Thr Ala Thr His Val Pro Ser Phe Glu Pro
    690                 695                 700

tga 2115

<210> 4
<211> 704
<212> PRT
<213> Arabidopsis thaliana

<400> 4

Met Ala Thr Cys Ser Ser Ser Leu Leu Val Leu Pro Asn Leu Arg Leu
1               5                   10                  15

Ser Ser Asn Gln Arg Arg Asn Phe Lys Val Arg Ala Gln Ile Ser Gly
            20                  25                  30

Glu Asn Lys Lys Ala Thr Ser Leu Glu Pro Val Asn Asn Asn Gly Ser
        35                  40                  45

Val Ser Leu Ser Thr Thr Val Gln Asn Gln Lys Gly Ala Asn Glu Val
    50                  55                  60

Asn Gly Lys Gly Lys Ser Lys Arg Lys Ile Val Ser Asp Glu Ile Glu
65                  70                  75                  80

Leu Leu Trp Asp Asp Gly Tyr Gly Ser Lys Ser Val Lys Asp Tyr Phe

                85                  90                  95

Ala Ala Ala Lys Glu Ile Leu Lys Ala Asp Gly Gly Pro Pro Arg Trp
            100                 105                 110

Phe Ser Pro Val Asp Cys Gly Arg Pro Val Glu Asp Ala Pro Thr Leu
        115                 120                 125

Leu Phe Leu Pro Gly Met Asp Gly Thr Gly Met Gly Leu Val Pro His
    130                 135                 140

His Lys Ala Leu Gly Lys Ala Phe His Val Ser Cys Leu His Ile Pro
145                 150                 155                 160

Val Leu Asp Arg Thr Pro Phe Glu Gly Leu Leu Lys Val Val Glu Asp
                165                 170                 175

Val Leu Arg Gln Glu Gln Ala Thr Arg Pro Asn Lys Pro Ile Tyr Leu
            180                 185                 190

Val Gly Asp Ser Phe Gly Gly Cys Leu Ala Leu Ala Val Ala Ala Arg
        195                 200                 205

Asn Arg Ser Leu Asp Leu Val Leu Ile Leu Val Asn Pro Ala Thr Ser
    210                 215                 220

Phe Asp Arg Ser Pro Leu Gln Pro Leu Leu Pro Ile Leu Glu Met Val
225                 230                 235                 240

Pro Glu Glu Leu His Phe Thr Val Pro Tyr Ala Leu Ser Phe Ile Met
                245                 250                 255

Gly Asp Pro Ile Lys Met Ala Thr Leu Gly Ile Asp Asn Gln Leu Pro
            260                 265                 270

Thr Gly Val Lys Ile Glu Lys Leu Arg Gln Arg Leu Thr Lys Thr Met
        275                 280                 285

Leu Pro Leu Leu Ser Glu Leu Gly Gly Ile Ile Pro Arg Glu Thr Leu
    290                 295                 300

Leu Trp Lys Leu Lys Leu Leu Arg Ser Gly Cys Ala Tyr Ala Asn Ser
305                 310                 315                 320

Arg Ile His Ala Val Gln Ala Glu Val Leu Val Leu Ala Ser Gly Lys
                325                 330                 335

Asp Met Met Leu Pro Ser Gln Glu Glu Ala Lys Arg Leu His Gly Leu
            340                 345                 350

Leu Lys Asn Cys Ser Val Arg Cys Phe Lys Asp Asn Gly His Thr Leu
        355                 360                 365

Leu Leu Glu Asp Ser Ile Ser Leu Leu Thr Val Ile Lys Gly Thr Gly
    370                 375                 380

Lys Tyr Arg Arg Ser Trp Arg Tyr Asp Leu Val Ser Asp Phe Leu Pro
385                 390                 395                 400

Pro Ser Lys Gly Glu Leu Ala Tyr Ala Leu Asp Glu Val Leu Gly Phe
                405                 410                 415

Leu Arg Asn Ala Val Gly Ser Val Phe Phe Ser Thr Met Glu Asp Gly

            420                 425                 430

Lys Ile Val Lys Gly Leu Ala Gly Val Pro Asp Lys Gly Pro Val Leu
        435                 440                 445

Leu Val Gly Tyr His Met Leu Met Gly Leu Glu Leu Gly Pro Met Ser
    450                 455                 460

Glu Ala Phe Ile Lys Glu Lys Asn Ile Leu Phe Arg Gly Met Ala His
465                 470                 475                 480

Pro Val Leu Tyr Ser Asp Asn Asp Pro Ala Lys Ala Phe Asp Tyr Gly
                485                 490                 495

Asp Trp Ile Lys Val Phe Gly Ala Tyr Pro Val Thr Ala Thr Asn Leu
            500                 505                 510

Phe Lys Leu Leu Asp Ser Lys Ser His Val Leu Leu Phe Pro Gly Gly
        515                 520                 525

Ala Arg Glu Ala Leu His Asn Arg Gly Glu Gln Tyr Lys Leu Ile Trp
    530                 535                 540

Pro Glu Gln Gln Glu Phe Val Arg Met Ala Ala Arg Phe Gly Ala Thr
545                 550                 555                 560

Ile Val Pro Phe Gly Thr Val Gly Glu Asp Asp Ile Ala Glu Leu Val
                565                 570                 575

Leu Asp Tyr Asn Asp Leu Met Lys Ile Pro Ile Leu Asn Asp Tyr Ile
            580                 585                 590

Thr Glu Val Thr Arg Asp Thr Lys Gln Phe Lys Leu Arg Glu Glu Ser
        595                 600                 605

Glu Gly Glu Val Ala Asn Gln Pro Leu Tyr Leu Pro Gly Leu Ile Pro
    610                 615                 620

Lys Val Pro Gly Arg Phe Tyr Tyr Leu Phe Gly Lys Pro Ile Glu Thr
625                 630                 635                 640

Lys Gly Arg Pro Glu Leu Val Lys Asp Lys Glu Glu Ala Asn Gln Val
                645                 650                 655

Tyr Leu Glu Val Lys Ala Glu Val Glu Asn Ser Ile Ala Tyr Leu Leu
            660                 665                 670

Lys Lys Arg Glu Glu Asp Pro Tyr Arg Ser Val Leu Asp Arg Leu Asn
        675                 680                 685

Tyr Ser Leu Thr His Thr Thr Ala Thr His Val Pro Ser Phe Glu Pro
    690                 695                 700

<210> 5
<211> 688
<212> PRT
<213> Arabidopsis thaliana

<400> 5

Met Ala Val Pro Thr Leu Cys Ile Ala Asp Gly Ile Tyr Pro Thr Ala
1               5                   10                  15

Phe Phe Tyr Arg Asn Ser Val Arg Arg Leu Thr Ser Val Ala His Asp
            20                  25                  30

Arg Phe Thr Asn Pro Lys Phe Gly Ile Asp Gly Arg Leu Arg Asn Gly
        35                  40                  45

Pro Lys Val Thr Ala Thr Val Asn Pro Tyr Thr Tyr Thr Glu Ala Ala
    50                  55                  60

Gln Pro Glu Glu Arg Lys Ser Leu Thr Asp Phe Leu Val Glu Ala Arg
65                  70                  75                  80

Asp Phe Val Arg Ser Asp Gly Gly Asp Gly Gly Pro Pro Arg Trp Phe
                85                  90                  95

Ser Pro Leu Glu Cys Gly Ala Arg Ala Pro Glu Ser Pro Leu Leu Leu
            100                 105                 110

Tyr Leu Pro Gly Ile Asp Gly Thr Gly Leu Gly Leu Ile Arg Gln His
        115                 120                 125

Lys Arg Leu Gly Glu Ile Phe Asp Ile Trp Cys Leu His Phe Pro Val
    130                 135                 140

Thr Asp Arg Thr Pro Ala Arg Asp Leu Val Lys Leu Ile Glu Arg Thr
145                 150                 155                 160

Val Arg Ser Glu Tyr Phe Arg Leu Pro Lys Arg Pro Ile Tyr Ile Val
                165                 170                 175

Gly Glu Ser Ile Gly Ala Cys Leu Ala Leu Asp Val Ala Ala Ser Asn
            180                 185                 190

Pro Asp Ile Asp Leu Val Leu Ile Leu Ala Asn Pro Val Thr Arg Val
        195                 200                 205

Asn Asn Phe Met Leu Gln Pro Leu Ser Ser Leu Leu Glu Ile Leu Pro
    210                 215                 220

Asp Gly Val Pro Ser Phe Leu Glu Glu Asn Phe Arg Phe Glu Gln Gly
225                 230                 235                 240

Tyr Pro Phe Ala Ala Met Phe Glu Thr Met Leu Asn Glu Thr Asp Ala
                245                 250                 255

Ala Gln Ile Gly Gly Gly Leu Leu Gly Asp Leu Phe Ala Thr Ser Val
            260                 265                 270

Asn Leu Pro Thr Leu Ala Arg Ile Phe Pro Lys Asp Thr Leu Leu Trp
        275                 280                 285

Lys Leu Gln Leu Leu Lys Ser Ala Ser Ala Ser Ala Lys Ser His Met
    290                 295                 300

Tyr Thr Val Lys Ala Gln Thr Leu Ile Leu Leu Ser Gly Arg Asp Gln
305                 310                 315                 320

Trp Leu Leu Asn Lys Glu Asp Ile Glu Lys Leu His Cys Thr Leu Pro
                325                 330                 335

Asn Cys Glu Val Arg Lys Phe Glu Asn Tyr Gly Gln Leu Leu Phe Leu
            340                 345                 350

Glu Asp Gly Val Asp Leu Val Thr Ile Ile Lys Cys Thr Tyr Tyr Tyr
        355                 360                 365

Arg Arg Gly Lys Leu Leu Asp Tyr Val Ser Asp Phe Ile Leu Pro Thr
    370                 375                 380
Pro Phe Glu Leu Lys Glu Tyr Glu Glu Ser Gln Arg Leu Leu Thr Ala
385                 390                 395                 400
Ile Thr Ser Pro Val Phe Leu Ser Thr Leu Asp Asn Gly Thr Val Val
                405                 410                 415
Arg Ser Leu Ala Gly Ile Pro Ser Glu Gly Pro Val Leu Tyr Val Gly
            420                 425                 430
Asn His Met Leu Leu Gly Thr Glu Leu Arg Pro Ala Ala Ile His Phe
        435                 440                 445
Leu Lys Glu Lys Asn Ile Leu Leu Arg Gly Leu Ala His Pro Val Met
    450                 455                 460
Phe Ala Lys Lys Tyr Gly Ser Lys Leu Pro Asp Met His Met Phe Asp
465                 470                 475                 480
Ser Val Arg Met Ile Gly Ala Val Pro Val Ser Asn Ile Asn Phe Tyr
                485                 490                 495
Lys Leu Leu Arg Ser Lys Ala His Val Val Leu Tyr Pro Gly Gly Val
            500                 505                 510
Arg Glu Ala Leu His Arg Lys Gly Glu Val Tyr Lys Leu Phe Trp Pro
        515                 520                 525

Glu His Ser Glu Phe Val Arg Thr Ala Ser Lys Phe Gly Thr Lys Ile
    530                 535                 540

Ile Pro Phe Gly Val Val Gly Glu Asp Asp Leu Cys Glu Val Val Phe
545                 550                 555                 560

Asp Tyr Asn Asp Gln Met Lys Ile Pro Phe Leu Lys Asn Leu Ile Lys
                565                 570                 575

Glu Leu Ser Gln Asp Ser Thr Tyr Leu Arg Asn Gly Glu Glu Gly Glu
            580                 585                 590

Val Gly Asn Gln Asp Leu His Met Pro Gly Ile Val Pro Lys Met Pro
        595                 600                 605

Gly Arg Phe Tyr Val Tyr Phe Gly Lys Pro Ile Tyr Thr Glu Gly Arg
    610                 615                 620

Glu Asp Glu Leu Asn Asp Lys Glu Lys Ala His Glu Val Tyr Leu Gln
625                 630                 635                 640

Val Lys Ser Glu Val Glu Arg Cys Met Thr Tyr Leu Lys Ile Lys Arg
                645                 650                 655

Glu Gly Asp Pro Tyr Arg Asn Ile Leu Ala Arg Ser Leu Tyr His Phe
            660                 665                 670

Ser His Gly Phe Ser Ser Gln Val Pro Thr Phe Asp Leu Arg Asn Gln
        675                 680                 685

<210> 6
<211> 684

<212>  PRT
<213>  Arabidopsis thaliana

<400>  6

Met Ala Ile Ile Thr Thr Thr Thr Val Arg Phe Thr Asp Gly Thr Ser
1               5                   10                  15

Pro Thr Phe Phe Ser Ser Ala Ser Thr Lys Ala Tyr Asn Leu His Phe
            20                  25                  30

Leu Tyr Ser Asn Ser Thr Gln Arg Leu Thr Asn Pro Lys Phe Gly Ile
        35                  40                  45

Gly Gly Lys Leu Lys Val Thr Val Asn Pro Tyr Ser Tyr Thr Glu Glu
    50                  55                  60

Val Arg Pro Glu Glu Arg Lys Ser Leu Thr Asp Phe Leu Thr Glu Ala
65                  70                  75                  80

Gly Asp Phe Val Asn Ser Asp Gly Gly Asp Gly Gly Pro Pro Arg Trp
                85                  90                  95

Phe Ser Pro Leu Glu Cys Gly Ala Arg Ala Pro Glu Ser Pro Leu Leu
            100                 105                 110

Leu Tyr Leu Pro Gly Ile Asp Gly Thr Gly Leu Gly Leu Ile Arg Gln
        115                 120                 125

His Lys Arg Leu Gly Glu Ile Phe Asp Ile Trp Cys Leu His Phe Pro
    130                 135                 140

Val Lys Asp Arg Thr Pro Ala Arg Asp Ile Gly Lys Leu Ile Glu Lys

145                 150                 155                 160

Thr Val Arg Ser Glu His Tyr Arg Phe Pro Asn Arg Pro Ile Tyr Ile
                165                 170                 175

Val Gly Glu Ser Ile Gly Ala Ser Leu Ala Leu Asp Val Ala Ala Ser
            180                 185                 190

Asn Pro Asp Ile Asp Leu Val Leu Ile Leu Ala Asn Pro Val Thr Arg
        195                 200                 205

Phe Thr Asn Leu Met Leu Gln Pro Val Leu Ala Leu Leu Glu Ile Leu
    210                 215                 220

Pro Asp Gly Val Pro Gly Leu Ile Thr Glu Asn Phe Gly Phe Tyr Gln
225                 230                 235                 240

Glu Met Phe Glu Thr Met Leu Asn Glu Asn Asp Ala Ala Gln Met Gly
                245                 250                 255

Arg Gly Leu Leu Gly Asp Phe Phe Ala Thr Ser Ser Asn Leu Pro Thr
            260                 265                 270

Leu Ile Arg Ile Phe Pro Lys Asp Thr Leu Leu Trp Lys Leu Gln Leu
        275                 280                 285

Leu Lys Ser Ala Ser Ala Ser Ala Asn Ser Gln Met Asp Thr Val Asn
    290                 295                 300

Ala Gln Thr Leu Ile Leu Leu Ser Gly Arg Asp Gln Trp Leu Met Asn
305                 310                 315                 320

Lys Glu Asp Ile Glu Arg Leu Arg Gly Ala Leu Pro Arg Cys Glu Val
                325                 330                 335

Arg Glu Leu Glu Asn Asn Gly Gln Phe Leu Phe Leu Glu Asp Gly Val
            340                 345                 350

Asp Leu Val Ser Ile Ile Lys Arg Ala Tyr Tyr Tyr Arg Arg Gly Lys
        355                 360                 365

Ser Leu Asp Tyr Ile Ser Asp Tyr Ile Leu Pro Thr Pro Phe Glu Phe
    370                 375                 380

Lys Glu Tyr Glu Glu Ser Gln Arg Leu Leu Thr Ala Val Thr Ser Pro
385                 390                 395                 400

Val Phe Leu Ser Thr Leu Lys Asn Gly Ala Val Val Arg Ser Leu Ala
                405                 410                 415

Gly Ile Pro Ser Glu Gly Pro Val Leu Tyr Val Gly Asn His Met Leu
            420                 425                 430

Leu Gly Met Glu Leu His Ala Ile Ala Leu His Phe Leu Lys Glu Arg
        435                 440                 445

Asn Ile Leu Leu Arg Gly Leu Ala His Pro Leu Met Phe Thr Lys Lys
    450                 455                 460

Thr Gly Ser Lys Leu Pro Asp Met Gln Leu Tyr Asp Leu Phe Arg Ile
465                 470                 475                 480

Ile Gly Ala Val Pro Val Ser Gly Met Asn Phe Tyr Lys Leu Leu Arg

                485                 490                 495

Ser Lys Ala His Val Ala Leu Tyr Pro Gly Gly Val Arg Glu Ala Leu
            500                 505                 510

His Arg Lys Gly Glu Glu Tyr Lys Leu Phe Trp Pro Glu His Ser Glu
        515                 520                 525

Phe Val Arg Ile Ala Ser Lys Phe Gly Ala Lys Ile Ile Pro Phe Gly
    530                 535                 540

Val Val Gly Glu Asp Asp Leu Cys Glu Met Val Leu Asp Tyr Asp Asp
545                 550                 555                 560

Gln Met Lys Ile Pro Phe Leu Lys Asn Leu Ile Glu Glu Ile Thr Gln
                565                 570                 575

Asp Ser Val Asn Leu Arg Asn Asp Glu Glu Gly Glu Leu Gly Lys Gln
            580                 585                 590

Asp Leu His Leu Pro Gly Ile Val Pro Lys Ile Pro Gly Arg Phe Tyr
        595                 600                 605

Ala Tyr Phe Gly Lys Pro Ile Asp Thr Glu Gly Arg Glu Lys Glu Leu
    610                 615                 620

Asn Asn Lys Glu Lys Ala His Glu Val Tyr Leu Gln Val Lys Ser Glu
625                 630                 635                 640

Val Glu Arg Cys Met Asn Tyr Leu Lys Ile Lys Arg Glu Thr Asp Pro
                645                 650                 655

Tyr Arg Asn Ile Leu Pro Arg Ser Leu Tyr Tyr Leu Thr His Gly Phe
            660                 665                 670

Ser Ser Gln Ile Pro Thr Phe Asp Leu Arg Asn His
        675                 680

<210> 7
<211> 701
<212> PRT
<213> Arabidopsis thaliana

<400> 7

Met Ala Val Thr Val Leu Pro Ser Val Ser Gly Leu Ser Ala Val Ala
1               5                   10                  15

Ser Ser Ser Asn Leu Arg Arg Leu Thr Ser Ala Ser Asn His Arg Leu
            20                  25                  30

Thr Ala Ile Lys Ser Val Thr Ser Thr Ser Ser Pro Pro Thr Pro Ser
        35                  40                  45

Ser Gly Val Gln Arg Arg Arg Lys Asn Asn Asp Glu Asn Arg Ala Thr
    50                  55                  60

Val Ala Lys Val Val Glu Asn Pro Tyr Ser Lys Val Glu Ala Ala Arg
65                  70                  75                  80

Pro Asp Leu Gln Lys Arg Leu Ser Asp Phe Leu Glu Glu Ala Arg Glu
                85                  90                  95

Phe Val Gly Asp Gly Gly Gly Pro Pro Arg Trp Phe Ser Pro Leu Glu
            100                 105                 110

Cys Gly Ala Gln Ala Thr Asn Ser Pro Leu Leu Leu Tyr Leu Pro Gly
        115                 120                 125

Ile Asp Gly Thr Gly Leu Gly Leu Ile Arg His His Lys Lys Leu Gly
    130                 135                 140

Glu Ile Phe Asp Ile Trp Cys Leu His Ile Pro Val Ser Asp Arg Thr
145                 150                 155                 160

Pro Val Lys Asp Leu Val Lys Leu Ile Glu Glu Thr Val Lys Ser Glu
                165                 170                 175

Asn Phe Arg Leu Pro Asn Arg Pro Ile Tyr Leu Val Gly Glu Ser Ile
            180                 185                 190

Gly Ala Cys Leu Ala Leu Asp Val Ala Ala Arg Asn Pro Asn Ile Asp
        195                 200                 205

Leu Ser Leu Ile Leu Val Asn Pro Ala Thr His Val Asn Asn Phe Met
    210                 215                 220

Val Gln Pro Leu Ser Gly Met Leu Asn Val Leu Pro Asp Gly Leu Pro
225                 230                 235                 240

Thr Leu Leu Glu Asp Ile Phe Asp Phe Gly Phe Lys Gln Gly Asp Pro
                245                 250                 255

Leu Thr Gly Met Leu Asp Ala Leu Ser Asn Glu Phe Ser Val Gln Arg
            260                 265                 270

Met Gly Gly Val Gly Gly Gly Met Leu Arg Asp Val Leu Ala Val Ser
        275                 280                 285

Ala Asn Leu Pro Thr Leu Ser Arg Met Phe Pro Lys Asp Thr Leu Leu
    290                 295                 300

Trp Lys Leu Glu Met Leu Lys Tyr Ala Ile Ala Ser Val Asn Ser His
305                 310                 315                 320

Ile Tyr Ser Val Arg Ala Glu Thr Leu Ile Leu Leu Ser Gly Arg Asp
                325                 330                 335

His Trp Leu Leu Lys Glu Glu Asp Ile Asp Arg Tyr Ser Arg Thr Leu
            340                 345                 350

Pro Lys Cys Ile Val Arg Lys Leu Asp Asp Asn Gly Gln Phe Pro Leu
        355                 360                 365

Leu Glu Asp Gly Val Asp Leu Ala Thr Ile Ile Lys Cys Thr Cys Phe
    370                 375                 380

Tyr Arg Arg Gly Lys Ser His Asp His Ile Thr Asp Tyr Ile Met Pro
385                 390                 395                 400

Thr Thr Phe Glu Leu Lys Gln Gln Val Asp Asp His Arg Leu Leu Met
                405                 410                 415

Asp Gly Thr Ser Pro Val Met Leu Ser Thr Leu Glu Asp Gly Thr Val
            420                 425                 430

Val Arg Ser Leu Glu Gly Leu Pro Ser Glu Gly Pro Val Leu Tyr Val
        435                 440                 445

Gly Tyr His Met Ile Leu Gly Phe Glu Leu Ala Pro Met Val Ile Gln
    450                 455                 460

Leu Met Thr Glu Arg Asn Ile His Leu Arg Gly Leu Ala His Pro Met
465                 470                 475                 480

Leu Phe Lys Asn Leu Gln Asp Ser Leu Val Asp Thr Lys Met Phe Asp
                485                 490                 495

Lys Tyr Lys Ile Met Gly Gly Val Pro Val Ser His Phe Asn Ile Tyr
            500                 505                 510

Lys Leu Leu Arg Glu Lys Ala His Val Leu Leu Tyr Pro Gly Gly Val
        515                 520                 525

Arg Glu Ala Leu His Arg Lys Gly Glu Glu Tyr Lys Leu Phe Trp Pro
    530                 535                 540

Glu Arg Ser Glu Phe Val Arg Val Ala Ser Lys Phe Gly Ala Lys Ile
545                 550                 555                 560

Val Pro Phe Gly Val Val Gly Glu Asp Asp Ile Cys Glu Ile Val Leu
                565                 570                 575

Asp Ser Asn Asp Gln Arg Asn Ile Pro Ile Leu Lys Asp Leu Met Glu
            580                 585                 590

Lys Ala Thr Lys Asp Ala Gly Asn Ile Arg Glu Gly Asp Glu Ser Glu
        595                 600                 605

Leu Gly Asn Gln Glu Cys Tyr Phe Pro Gly Leu Val Pro Lys Ile Pro
    610                 615                 620

Gly Arg Phe Tyr Tyr Tyr Phe Gly Lys Pro Ile Glu Thr Ala Gly Lys
625                 630                 635                 640

Glu Lys Glu Leu Lys Asp Lys Glu Lys Ala Gln Glu Leu Tyr Leu Gln
                645                 650                 655

Val Lys Ser Glu Val Glu Gln Cys Ile Asp Tyr Leu Lys Val Lys Arg
            660                 665                 670

Glu Ser Asp Pro Tyr Arg His Leu Leu Pro Arg Met Leu Tyr Gln Ala
        675                 680                 685

Ser His Gly Trp Ser Ser Glu Ile Pro Thr Phe Asp Leu
    690                 695                 700

<210> 8
<211> 692
<212> PRT
<213> Hordeum vulgare

<400> 8

Met Ser Met Val Pro His Ile Met Leu Arg Pro Phe Gly Val Thr Pro
1               5                   10                  15

Cys Gly Gly Gly Gly Gly Gly Cys Leu Arg Arg His His Ala Arg Leu
            20                  25                  30

Cys Leu Pro Arg Ala Ser Ser Val Asp Ala Thr Gly Gly Ser Glu Pro
        35                  40                  45

Gly Asp Gly Ser Asn Arg Arg Arg Arg Lys Lys Gln Ala Ala Glu Glu
50                  55                  60

Pro Arg Leu Glu Val Leu Tyr Asp Asp Gly Phe Gly Ser Val Thr Met
65                  70                  75                  80

Lys Asp Tyr Trp Glu Ala Val Arg Ala Met Pro Lys Asp Asp Gly Gly
                85                  90                  95

Pro Pro Arg Trp Phe Cys Pro Val Glu Cys Gly Arg Pro Glu Val Asp
            100                 105                 110

Arg Ser Pro Leu Leu Leu Val Leu Pro Gly Thr Asp Gly Val Gly Met
        115                 120                 125

Glu Leu Ile Leu His His Lys Ser Leu Gly Lys Val Phe Glu Val Cys
    130                 135                 140

Cys Phe His Ile Pro Leu Ser Asp Arg Thr Pro Phe Glu Gly Leu Leu
145                 150                 155                 160

Gln Ile Val Glu Glu Tyr Val Lys Tyr Glu Ser Ala Leu Ser Pro Ser
                165                 170                 175

Arg Pro Ile Tyr Ile Ile Gly Asp Ser Phe Gly Gly Cys Leu Ala Ile
            180                 185                 190

Ser Val Ala Ala Arg Asn Pro Glu Ile Asp Leu Val Leu Thr Leu Val
        195                 200                 205

Asn Pro Ala Thr Ser Ser Ala Lys Thr Ser Leu Gln Ala Val Leu Pro

210                 215                 220

Leu Leu Glu Ala Val Pro Ser Asp Leu Pro Val Val His Pro His Leu
225                 230                 235                 240

Leu Lys Tyr Leu Ile Gly Asn Pro Leu Asn Val Ala Met Val Ser Val
                245                 250                 255

Gln Asn Gly Leu Ser Pro Gln Glu Thr Leu Gln Glu Phe Ser Asn Ser
            260                 265                 270

Leu Ala Ser Met Leu Pro Leu Val Ser Glu Leu Gly Asp Ile Ile Gln
        275                 280                 285

Met Gly Thr Leu Val Trp Lys Leu Lys Leu Leu Lys Ser Gly Ala Asn
    290                 295                 300

Tyr Ala Asn Ser Gln Leu Cys Ala Val Gln Ala Glu Val Leu Leu Leu
305                 310                 315                 320

Ala Ser Gly Ile Glu Asn Leu Pro Pro Ser Gly Glu Ala His Arg Leu
                325                 330                 335

Phe Lys Thr Leu Lys Asn Cys Lys Leu Arg Tyr Phe Arg Asn Arg Gly
            340                 345                 350

Asp Arg Leu Leu Met Glu Asp Gly Phe Asn Leu Leu Thr Val Ile Lys
        355                 360                 365

Gly Val Asn Met Tyr Arg Arg Gly Arg Gln Arg Asp Pro Val Asn Asp
    370                 375                 380

Phe Leu Ser Pro Thr Leu Ser Glu Phe Lys Arg Thr Phe Gly Glu Asp
385                 390                 395                 400

Phe Lys Leu Phe Asn Gln Leu Leu Ser Pro Val Met Leu Ser Thr Leu
                405                 410                 415

Lys Asn Gly Asn Ile Val Arg Gly Leu Ala Gly Val Pro Asp Lys Gly
            420                 425                 430

Pro Val Leu Phe Val Gly Tyr His Gln Leu Phe Ala Met Glu Val Leu
        435                 440                 445

Ala Leu Ile Glu Gly Phe Leu Arg Glu Lys Lys Thr Ile Leu Arg Thr
    450                 455                 460

Thr Ala His Gln Val Phe Phe Val Gly Asn Tyr Glu Thr Leu Arg Gln
465                 470                 475                 480

Glu Leu Ser Leu Phe Asp Trp Phe Ser Thr Tyr Gly Ala Leu Pro Val
                485                 490                 495

Ser Pro Ile Asn Thr Tyr Lys Leu Phe Glu Arg Asn Glu Phe Val Leu
            500                 505                 510

Leu Tyr Pro Gly Gly Val Arg Glu Ala Leu His Arg Lys Gly Glu Ala
        515                 520                 525

Tyr Lys Leu Phe Trp Pro Asp Gln Pro Glu Phe Val Arg Met Ala Ala
    530                 535                 540

Arg Phe Gly Val Thr Val Ile Pro Phe Gly Cys Val Gly Glu Asp Asp

545                 550                 555                 560

Phe Met Glu Val Val Val Asp Tyr Asn Asp Gln Lys Asn Ile Pro Tyr
                565                 570                 575

Leu Lys Asp Glu Ile Lys Ser Phe Asn Glu Asp Phe Thr Gly Ile Ile
            580                 585                 590

Arg Asp Thr Val Lys Gly Asp Asp Gly Asn Gln Val Leu His Val Pro
        595                 600                 605

Ala Val Leu Pro Lys Val Pro Gly Arg Leu Tyr Phe Leu Phe Gly Lys
    610                 615                 620

Pro Ile Glu Met Lys Gly Met Asp Asn Val Leu Thr Asp Arg Lys Lys
625                 630                 635                 640

Ala Asn Glu Val Tyr Leu Gln Ile Glu Ser Glu Val Glu Asn Leu Met
                645                 650                 655

Ser Tyr Leu Lys Arg Lys Arg Ser Glu Asp Pro Tyr Arg Ser Ile Thr
            660                 665                 670

Arg Arg Ala Leu Tyr Gln Ala Thr Arg Gly Pro Ser Thr Gln Val Pro
        675                 680                 685

Thr Phe Glu Pro
    690

<210>  9
<211>  682
<212>  PRT

<213> Oryza sativa

<400> 9

Met Ser Met Ser Ile Ala Leu His Ala Val Leu Pro Pro Ala Ala Ala
1               5                   10                  15

Thr Pro Arg Arg Arg Pro Thr Arg Leu Arg Ala Ser Ser Thr Glu Ala
            20                  25                  30

Pro Ala Ser Gly Glu Lys Gly Gln Glu Asp Thr Asp Arg Lys Ser Gly
        35                  40                  45

Thr Gly Met Arg Arg Arg Arg Arg Arg Ala Lys Lys Val Gln Glu Val
    50                  55                  60

Gly Leu Glu Ala Leu Tyr Asp Asp Gly Phe Gly Glu Ala Thr Val Arg
65                  70                  75                  80

Asp Tyr Phe Asp Ala Leu Arg Ala Thr Pro Leu Asp Gly Gly Gly Gly
                85                  90                  95

Gly Gly Pro Pro Ala Val His Ala Pro Pro Leu Leu Leu Phe Leu Pro
            100                 105                 110

Gly Ile Asp Gly Val Gly Met Glu Leu Ile Met Gln His Lys Ser Leu
        115                 120                 125

Gly Lys Val Phe Glu Val Arg Cys Leu His Ile Pro Val Asn Asp Arg
    130                 135                 140

Thr Pro Tyr Glu Gly Leu Leu Gln Ile Met Glu Glu Ser Val Lys Tyr
145                 150                 155                 160

Glu His Asn Leu Ser Pro Asn Arg Pro Ile Tyr Ile Ile Gly Asp Ser
                165                 170                 175

Phe Gly Gly Cys Leu Ala Leu Ser Leu Ala Ser Arg Asn Pro Glu Ile
            180                 185                 190

Asp Leu Val Leu Ile Leu Val Asn Pro Ala Thr Ser Phe Ala Lys Thr
        195                 200                 205

Pro Leu Gln Ala Ile Leu Pro Leu Leu Glu Met Val Pro Ser Asn Leu
    210                 215                 220

Pro Val Thr Leu Pro His Leu Leu Arg Tyr Leu Ile Gly Asp Pro Leu
225                 230                 235                 240

Lys Met Ala Met Val Ser Ile Gln Asn Asn Thr Ser Pro Gln Asp Thr
                245                 250                 255

Leu Glu Ser Phe Ser Asp Ser Leu Ser Ser Met Leu Pro Leu Leu Ser
            260                 265                 270

Glu Phe Gly His Ile Val Arg Met Asp Thr Leu Val Trp Lys Leu Lys
        275                 280                 285

Leu Leu Met Ser Gly Val Asp Tyr Thr Asn Ser Arg Leu Asn Ala Val
    290                 295                 300

Gln Ala Glu Ile Leu Leu Leu Ala Ser Gly Asn Asp Asn Leu Pro Pro
305                 310                 315                 320

Ser Gly Glu Ala Asp Arg Leu Phe Lys Ala Leu Lys Ser Cys Lys Val
                325                 330                 335

Arg Tyr Phe Arg Thr Ser Ser Asp Arg Leu Leu Met Glu Ser Ser Phe
            340                 345                 350

Asn Leu Leu Thr Val Ile Lys Gly Ala Ser Met Tyr Arg Gln Gly Lys
        355                 360                 365

Gln Arg Asp Thr Ile Thr Asp Phe Leu Pro Pro Thr Ile Ser Glu Phe
    370                 375                 380

Lys Arg Thr Phe Gly Glu Asp Phe Lys Leu Leu His His Leu Leu Ser
385                 390                 395                 400

Pro Val Met Leu Ser Thr Leu Arg Asn Gly Lys Ile Val Arg Gly Leu
                405                 410                 415

Ala Gly Val Pro Asp Lys Gly Pro Val Leu Leu Val Gly Tyr His Gln
            420                 425                 430

Leu Leu Ala Met Glu Ile Thr Ser Met Ala Glu Glu Phe Leu Arg Glu
        435                 440                 445

Lys Lys Ala Val Leu Arg Thr Leu Ala His Pro Val Phe Phe Val Gly
    450                 455                 460

Asn Tyr Glu Ile Leu Arg Gln Glu Leu Ser Phe Phe Asp Val Val Pro
465                 470                 475                 480

Leu Tyr Gly Gly Val Gln Val Ser Pro Ile Asn Thr Tyr Arg Leu Phe
                485                 490                 495

Glu Arg Asp Glu Phe Val Leu Leu Tyr Pro Gly Gly Ile Arg Glu Ala
            500                 505                 510

Leu His Arg Lys Asp Glu Asp Tyr Gln Leu Phe Trp Pro Asp Gln Pro
        515                 520                 525

Glu Phe Val Arg Met Ala Ala Gln Phe Gly Val Thr Val Ile Pro Phe
    530                 535                 540

Gly Cys Val Gly Glu Asp Asp Met Leu Glu Ile Val Leu Asp Tyr Asn
545                 550                 555                 560

Glu Leu Lys Asn Ile Pro Tyr Ile Arg Glu Thr Ile Glu Ser Phe Asn
                565                 570                 575

Gln Asp Cys Pro Gly Val Arg Ser Thr Val Lys Gly Glu Glu Gly Asn
            580                 585                 590

Gln Val Leu His Leu Pro Ala Val Leu Pro Lys Leu Pro Gly Arg Leu
        595                 600                 605

Tyr Tyr Leu Phe Gly Lys Pro Ile Glu Met Lys Gly Met Asp Gly Val
    610                 615                 620

Gln Arg Asp Arg Glu Ser Ala Asn Gln Leu Tyr Leu Asp Ile Lys Ser
625                 630                 635                 640

Glu Val Glu Asn Ile Met Ser Tyr Leu Lys Arg Lys Arg Glu Gln Asp
                645                 650                 655

Pro Tyr Arg Ser Ile Thr Ala Arg Thr Phe Tyr Gln Ala Thr Trp Gly
            660                 665                 670

Val Thr Ala Gln Ile Pro Thr Phe Glu Pro
        675                 680

<210> 10
<211> 698
<212> PRT
<213> Oryza sativa

<400> 10

Met Ser Ala Ala Leu His Thr Leu Pro Ala Val Ala Ala Gly Gly Leu
1               5                   10                  15

Gly Cys Arg Trp Arg Arg Pro Ser Ala Thr Leu Arg Ala Ala Ser Ser
            20                  25                  30

Val Gly Thr Ala Ser Val Ala Val Ser Val Asn Ala Glu Thr Gln Gln
        35                  40                  45

Gln Arg Gln Val Gly Val Glu Glu Glu Glu Lys Arg Arg Glu Asp Ala
    50                  55                  60

Ala Glu Val Arg Thr Gly Arg Val Val Glu Ala Leu Tyr Asp Asp Gly
65                  70                  75                  80

Phe Gly Gly Val Thr Val Lys Asp Tyr Phe Ala Ala Ala Arg Ala Val
                85                  90                  95

Ser Ser Asp Asp Gly Gly Pro Pro Arg Trp Phe Cys Pro Val Asp Ala
            100                 105                 110

Gly Arg Pro Ala Val Asp Asn Ala Pro Leu Leu Leu Phe Leu Pro Gly
        115                 120                 125

Thr Asp Gly Val Gly Met Gly Leu Ile Leu His His Lys Ser Leu Gly
    130                 135                 140

Arg Val Phe Glu Val Arg Cys Leu His Ile Pro Val Asn Asp Arg Thr
145                 150                 155                 160

Pro Phe Glu Gly Leu Leu Gln Ile Val Glu Asn Ser Ile Lys Tyr Glu
                165                 170                 175

His Ala Met Ser Pro Asn Arg Pro Ile Tyr Leu Ile Gly Asp Ser Phe
            180                 185                 190

Gly Gly Cys Leu Ala Leu Ser Val Ala Ala Arg Asn Pro Gln Ile Asp
        195                 200                 205

Leu Val Leu Ile Leu Ile Asn Pro Ala Thr Ser Phe Ala Lys Thr Pro
    210                 215                 220

Leu Gln Pro Ile Leu Pro Val Leu Glu Ala Met Pro Ser Glu Leu His
225                 230                 235                 240

Val Thr Val Pro Tyr Leu Leu Ser Phe Val Met Gly Asp Pro Leu Lys
                245                 250                 255

Met Ala Met Val Ser Val Glu Asn Asn Leu Ser Pro Pro Lys Thr Leu
            260                 265                 270

Gln Lys Leu Ser Asp Ser Leu Thr Ser Met Leu Pro Leu Leu Ser Glu

        275                 280                 285

Leu Ala Asp Ile Ile Pro Arg Asp Thr Leu Phe Trp Lys Leu Lys Leu
    290                 295                 300

Leu Lys Ser Gly Ala Ala Tyr Ala Asn Ser Arg Leu His Ala Val Gln
305                 310                 315                 320

Ala Glu Val Leu Leu Leu Ala Ser Gly Asn Asp Asn Leu Leu Pro Ser
                325                 330                 335

Gly Glu Glu Ala Asp Arg Leu Phe Lys Ser Leu Lys Asn Cys Arg Val
            340                 345                 350

Arg Tyr Phe Lys Asp Asn Gly His Thr Leu Leu Leu Glu Asp Gly Val
        355                 360                 365

Asn Leu Leu Ser Val Ile Lys Gly Val Asn Met Tyr Arg Arg Gly Arg
    370                 375                 380

Gln Arg Asp Pro Val Thr Asp Tyr Ile Pro Pro Thr Leu Ser Glu Phe
385                 390                 395                 400

Lys Lys Thr Phe Asp Glu Asp His Lys Leu Phe His Leu Ala Leu Ser
                405                 410                 415

Pro Val Met Leu Ser Thr Leu Lys Asn Gly Lys Ile Val Arg Gly Leu
            420                 425                 430

Thr Gly Val Pro Asp Gln Gly Pro Val Leu Phe Val Gly Tyr His Ala
        435                 440                 445

Leu Met Gly Ile Glu Leu Ser Pro Leu Tyr Glu Glu Phe Leu Arg Glu
450                 455                 460

Lys Arg Thr Ser Phe Arg Gly Met Ala His Pro Ile Leu Phe Gly Gly
465                 470                 475                 480

Lys His Glu Ser Ser Arg Gln Glu Leu Ser Arg Phe Asp Thr Ile Ser
                485                 490                 495

Met Tyr Gly Gly Leu Pro Val Thr Ala Ile Asn Met Tyr Arg Leu Phe
            500                 505                 510

Glu Arg Asn Gln Phe Val Leu Leu Tyr Pro Gly Gly Val Arg Glu Ala
        515                 520                 525

Leu His Arg Lys Gly Glu Ala Tyr Lys Leu Phe Trp Pro Asp Gln Pro
    530                 535                 540

Glu Phe Val Arg Met Ala Ala Arg Phe Gly Val Thr Ile Ile Pro Phe
545                 550                 555                 560

Gly Phe Val Gly Glu Asp Asp Val Leu Glu Leu Val Ala Asp Tyr Asn
                565                 570                 575

Asp Gln Lys Asn Ile Pro Tyr Leu Arg Glu Trp Ile Glu Ser Ile Asn
            580                 585                 590

Arg Glu Ala Gln Arg Val Arg Asp Ser Val Lys Gly Glu Asp Gly Asn
        595                 600                 605

Gln Asp Val His Ile Pro Ala Leu Leu Pro Lys Val Pro Gly Arg Phe

610                 615                 620

Tyr Tyr Leu Phe Gly Lys Pro Ile Glu Met Lys Gly Met Asp Asn Val
625                 630                 635                 640

Val Arg Asp Arg Lys Ser Ala Asn Glu Val Tyr Leu His Ile Lys Ser
                645                 650                 655

Glu Val Glu Ser Leu Met Ser Tyr Leu Lys Arg Lys Arg Glu Glu Asp
            660                 665                 670

Pro Tyr Arg Ser Ile Ala Gln Arg Ala Val Tyr Gln Ala Ser Trp Gly
        675                 680                 685

Ala Ser Ala Glu Val Pro Thr Phe Glu Pro
    690                 695

<210>  11
<211>  664
<212>  PRT
<213>  Oryza sativa

<400>  11

Met Ala Thr Leu Ser Leu Pro Leu His Ser Gln Ile Ala Leu Trp Arg
1               5                   10                  15

Gln Arg Arg Arg Arg His Asp Leu Phe Glu Arg Ile Ser Ser Cys His
            20                  25                  30

Gln Phe Lys His Ala Gly Trp Arg Leu Gln Val Ser Tyr Lys Gly Leu
        35                  40                  45

Glu Thr Leu Tyr Asp Asp Gly Tyr Gln Lys Ala Lys Asp Leu Asp Tyr
    50                  55                  60

Tyr Tyr Arg Ser Leu Gly Glu Leu Val Glu His Asp Ser Gly Pro Pro
65                  70                  75                  80

Arg Leu Phe Cys Pro Val Asp Ala Gly Ser Pro Ile Glu Asp Ala Pro
                85                  90                  95

Leu Met Leu Tyr Leu Pro Gly Val Asp Gly Met Gly Met Gly Leu Phe
            100                 105                 110

Met His His Lys Ala Leu Gly Arg Ile Phe Glu Leu Arg Cys Met His
        115                 120                 125

Ile Pro Phe His Asp Arg Thr Pro Phe Glu Glu Leu Val Glu Met Val
    130                 135                 140

Glu Asp Val Val Arg Ala Glu His Ala Thr Ser Pro Asn Lys Pro Ile
145                 150                 155                 160

Tyr Leu Leu Gly Thr Ser Phe Gly Gly Cys Ile Ala Leu Ala Val Ala
                165                 170                 175

Ala Arg Asn Pro Cys Ile Asp Leu Val Leu Val Leu Val Asn Pro Ala
            180                 185                 190

Thr Ser Phe Glu Lys Ser Asp Ile Lys Gln Leu Leu Ser Val Ser Ser
        195                 200                 205

Pro Leu Ser Asp Arg Ala Arg Ile Ala Ile Thr Ser Leu Leu Asn Tyr
    210                 215                 220

Asn Ile Asp Asn Glu Val Asp Met Ala Leu Ser Ser Met Lys Ser Gly
225                 230                 235                 240

Arg His Pro Leu Glu Ala Leu Asn Arg Leu Thr Ser Asn Ile Ser Ser
                245                 250                 255

Phe Leu Lys His Ser Asn Ile Leu Asn Lys Ile Pro Glu Asp Thr Leu
            260                 265                 270

Gly Trp Lys Met Lys Leu Ile Gln Gln Ala Ala Ser Tyr Ala Asn Cys
        275                 280                 285

Arg Leu Glu Ser Val Ser Ala Glu Val Leu Leu Leu Val Ser Cys Ala
    290                 295                 300

Asp Arg Leu Leu Pro Ser Lys Ser Glu Ala Asp Arg Leu Gln Arg Met
305                 310                 315                 320

Leu Pro Lys Cys Lys Val Phe Phe Phe Glu Asn His Gly His Ser Leu
                325                 330                 335

Leu Leu Glu Tyr Gly Val His Val Ser Ser Ile Ile Lys Cys Thr Ser
            340                 345                 350

Leu Tyr Arg His Ser Arg Arg Tyr His Arg Val Phe Asp Tyr Ile Pro
        355                 360                 365

Pro Ser Ala Thr Glu Leu Lys Glu Val Glu Lys Ala Gly Ser Asp Leu
    370                 375                 380

Arg Ala Arg Thr Cys Pro Ala Met Phe Ser Thr Met Gly Asp Gly Val
385                 390                 395                 400

Val Val Arg Gly Leu Ala Gly Val Pro Glu Glu Gly Pro Val Leu Leu
                405                 410                 415

Val Gly Asn His Met Leu Leu Gly Ile Glu Leu Ile Ser Leu Ala Thr
            420                 425                 430

Glu Phe Leu Arg Arg Lys Gly Arg Val Leu Arg Gly Ile Ala His Pro
        435                 440                 445

Leu Leu Phe Pro Asn Lys Thr Lys Thr Trp Ser Glu Gly His Asp Phe
    450                 455                 460

Phe Asp Phe Leu Asn Leu Trp Gly Gly Val Pro Met Thr Tyr Lys Tyr
465                 470                 475                 480

Ile Tyr Gln Leu Leu Ala Ala Gly Glu Phe Val Leu Leu Tyr Pro Gly
                485                 490                 495

Gly His Arg Glu Ala Leu His Cys Lys Gly Glu Glu His Arg Leu Phe
            500                 505                 510

Trp Pro Asp Gln Thr Glu Phe Val Arg Met Ala Ala Gln Phe Asn Ala
        515                 520                 525

Thr Ile Val Pro Phe Gly Val Val Gly Glu Asp Asp Leu Met Glu Leu
    530                 535                 540

Leu Cys Thr Phe Glu Asp Ile Arg Asn Ala Pro Phe Gly Lys Glu Ile
545                 550                 555                 560

Met Gln Ala Tyr Ser Asn His Leu Lys Leu Arg Asp Ile Asp His Glu
                565                 570                 575

Val Phe Phe Pro Gly Val Tyr Leu Lys Ile Pro Gly Arg Phe Tyr Tyr
            580                 585                 590

Arg Phe Gly Lys Pro Ile Pro Thr Lys Gly Met Gln Ala Val Met Thr
        595                 600                 605

Asp Lys Gln Ala Ala Gly Glu Leu Tyr Leu His Val Lys Ser Glu Val
    610                 615                 620

Lys Ala Met Ile Ala Tyr Leu Leu Glu Lys Arg Glu Glu Asp Lys Phe
625                 630                 635                 640

Arg Ser Ile Leu Pro Arg Ile Leu Tyr Gln Leu Gly Cys Gly His Asp
                645                 650                 655

Ser Glu Ile Pro Ser Phe Asp Pro
            660

<210>  12
<211>  665
<212>  PRT
<213>  Oryza sativa

<400>  12

Met Pro Met Pro Ala Thr Val Ala Ala Ala Pro Arg Pro Leu Leu Pro
1               5                   10                  15

Gly Leu Pro Arg Arg Gly Thr Ala Pro Pro Arg Gly Arg Trp Ser Ala

            20                  25                  30

Ser Ala Ala Ser Ala Ala Ser Arg Gly Val Ala Ala Lys Ala Ala Arg
        35                  40                  45

Arg Gly Val Arg Glu Tyr Val Glu Ala Ala Arg Glu Met Val Arg Arg
    50                  55                  60

Pro Asp Gly Gly Pro Ala Arg Trp Phe Ser Pro Leu Glu Cys Gly Gly
65                  70                  75                  80

Gly Gly Gly Arg Leu Pro Gly Ala Pro Thr Met Leu Tyr Leu Pro Gly
                85                  90                  95

Ile Asp Gly Val Gly Leu Gly Leu Ile Arg His His Glu Arg Leu Ala
            100                 105                 110

Lys Met Phe Asp Met Trp Cys Leu His Ile Pro Val Glu Asp Cys Thr
        115                 120                 125

Ser Phe Glu Gly Leu Val Glu Tyr Val Glu Ser Ala Val Lys Ser Glu
    130                 135                 140

Gly Gln Arg Ala Arg Asp Arg Pro Val Tyr Leu Val Gly Glu Ser Val
145                 150                 155                 160

Gly Ala Cys Ile Ala Leu Ala Val Ala Ala Arg Asn Pro Asp Ile Asp
                165                 170                 175

Leu Val Leu Ile Leu Val Asn Pro Gly Thr Ser Phe His Lys Ser Gln
            180                 185                 190

Leu Gln Ser Leu Ser Val Phe Leu Asp Leu Val Pro Glu Pro Phe His
        195                 200                 205

Leu Thr Thr Pro Gln Leu Leu Asn Phe Leu Thr Gly Asn Phe Met Lys
    210                 215                 220

Ile Pro Ser Thr Ile Val Gly Arg Gly Phe Ser Phe Gln Glu Ala Gly
225                 230                 235                 240

Gln Ala Leu Ser Glu Ile Thr Thr Ser Leu Leu Pro Ser Leu Met Ser
                245                 250                 255

Leu Leu Asp Val Leu Pro Lys Glu Ser Ile Val Trp Lys Leu Lys Met
            260                 265                 270

Leu Arg Thr Ala Ser Ser Phe Val Asn Ser Arg Leu His Ala Val Lys
        275                 280                 285

Ala Gln Thr Leu Val Leu Ala Ser Trp Asn Asp Glu Leu Leu Pro Ser
    290                 295                 300

Arg Glu Glu Ala Glu Arg Leu Arg Asp Ala Leu Glu Lys Cys Arg Ile
305                 310                 315                 320

Arg Asn Phe Lys Asp Asn Gly His Lys Ile Leu Leu Glu Ala Glu Phe
                325                 330                 335

Asp Leu Ala Thr Ala Ile Lys Gly Ala Gly Tyr Tyr Arg Arg Ser Leu
            340                 345                 350

Glu Thr Asp Phe Val Ser Asp Tyr Leu Pro Leu Thr Pro Asp Glu Phe

        355                 360                 365

Gln Lys Ala Thr Asp His Ile Arg Met Leu Gln Tyr Ile Ala Asn Pro
    370                 375                 380

Val Met Leu Ser Thr Leu Pro Asp Gly Lys Ile Val Arg Gly Leu Ser
385                 390                 395                 400

Gly Leu Pro Lys Gln Gly Pro Ala Val Ile Val Gly Tyr His Met Leu
                405                 410                 415

Leu Gly Phe Glu Leu Gly Pro Leu Val Thr Gly Val Leu Arg Ser Ser
            420                 425                 430

Gly Ile His Ile Arg Gly Leu Ala His Pro Phe Met Phe Asp Lys Lys
        435                 440                 445

Lys Glu Lys Ile Met Pro Asp Pro Ser Tyr Tyr Asp Met His Arg Ile
    450                 455                 460

Met Gly Ala Val Pro Val Thr Ala Gly Asn Phe Tyr Lys Leu Leu Ala
465                 470                 475                 480

Glu Lys His Phe Val Leu Leu Tyr Pro Gly Gly Ala Arg Glu Ala Leu
                485                 490                 495

His Arg Lys Gly Glu Glu Tyr Lys Leu Phe Trp Pro Glu Gln Ser Glu
            500                 505                 510

Phe Val Arg Met Ala Ser Arg Phe Gly Ala Thr Ile Ile Pro Phe Gly
        515                 520                 525

Val Val Gly Glu Asp Asp Ile Cys Asp Met Leu Leu Asp Tyr Asp Asp
    530                 535                 540

Leu Met Lys Ile Pro Phe Tyr Asp Ile Leu Asp Arg Met Leu Asn Glu
545                 550                 555                 560

Asp Gly Val Lys Leu Arg Thr Asp Ser Thr Gly Glu Leu Lys Tyr Gln
                565                 570                 575

Arg Ile His Pro Val Val Ala Ala Pro Lys Ile Pro Gly Arg Phe Tyr
            580                 585                 590

Phe Ile Phe Gly Lys Pro Ile Glu Thr Arg Gly Arg Glu Lys Glu Leu
        595                 600                 605

Arg Asp Lys Glu Asn Ala Gln His Leu Tyr Leu Asn Val Lys Ser Glu
    610                 615                 620

Val Glu Ser Cys Met Lys Tyr Leu Lys Glu Lys Arg Glu Lys Asp Pro
625                 630                 635                 640

Tyr Arg Asn Ile Leu Ala Arg Leu Leu Tyr Gln Met Val His Gly Leu
                645                 650                 655

Asp Ala Glu Val Pro Thr Phe Asp Pro
            660                 665

<210>  13
<211>  698
<212>  PRT
<213>  Brassica rapa

<400> 13

Met Ala Thr Thr Thr Thr Ser Ser Ser Leu Leu Ala Leu Pro Ser Phe
1               5                   10                  15

Arg Ser Asn Cys Asn Gln Arg Pro Ser Phe Lys Val Arg Ala Gln Ile
            20                  25                  30

Ser Gly Asp Lys Ala Thr Ser Val Glu Pro Val Asn Gly Ser Val Ser
        35                  40                  45

Val Ser Asn Leu Pro Asn Pro Lys Gly Ser Glu Val Asn Gly Lys Val
    50                  55                  60

Lys Phe Gln Arg Glu Ala Glu Ile Glu Leu Leu Trp Asp Asp Gly Tyr
65                  70                  75                  80

Gly Ser Lys Ser Val Lys Asp Tyr Phe Ala Ala Ala Arg Glu Ile Leu
                85                  90                  95

Lys Lys Pro Asp Gly Gly Pro Pro Arg Trp Phe Ser Pro Val Asp Cys
            100                 105                 110

Gly Gln Pro Ile Lys Asp Ala Pro Thr Leu Phe Phe Leu Pro Gly Met
        115                 120                 125

Asp Gly Thr Gly Met Gly Leu Val Pro His Gln Ile Ala Leu Gly Lys
    130                 135                 140

Ala Phe His Val Trp Cys Leu His Ile Pro Val His Asp Arg Thr Pro
145                 150                 155                 160

Phe Gln Gly Leu Leu Lys Ile Val Glu Asp Val Leu Arg Gln Glu His
                165                 170                 175

Ala Thr Arg Pro Asn Lys Pro Ile Tyr Leu Val Gly Asp Ser Phe Gly
            180                 185                 190

Gly Cys Met Ala Leu Ala Val Ala Ala Arg Asn Pro Ser Ile Asp Leu
        195                 200                 205

Val Leu Val Leu Val Asn Pro Ala Thr Ser Phe Glu Arg Ser Pro Leu
    210                 215                 220

Gln Pro Leu Leu Pro Ile Leu Glu Met Val Pro Glu Glu Leu His Phe
225                 230                 235                 240

Thr Ile Pro Tyr Ala Leu Ser Phe Ile Met Gly Asp Pro Ile Lys Met
                245                 250                 255

Ala Ser Ile Gly Ile Asp Asn Gln Leu Pro Ala Gly Val Lys Leu Asn
            260                 265                 270

Lys Leu Arg Glu Lys Leu Thr Asn Ser Met Leu Pro Leu Leu Ser Glu
        275                 280                 285

Leu Gly Gly Ile Ile Pro Arg Glu Thr Leu Leu Trp Lys Leu Lys Leu
    290                 295                 300

Leu Arg Ser Gly Ala Ala Tyr Thr Asn Ser Arg Ile His Ala Val Glu
305                 310                 315                 320

Ala Glu Val Leu Val Leu Phe Ser Gly Lys Asp Arg Met Leu Pro Ser
                325                 330                 335

Gln Glu Glu Ser Lys Arg Leu His Gly Leu Leu Lys Asn Cys Thr Val
            340                 345                 350

Arg Cys Phe Lys Glu Asn Gly His Thr Leu Leu Leu Glu Asp Ser Leu
        355                 360                 365

Ser Leu Leu Thr Val Ile Lys Gly Thr Cys Lys Tyr Arg Arg Ser Arg
    370                 375                 380

Arg Tyr Asp Phe Ala Ser Asp Phe Leu Pro Pro Ser Lys Gly Glu Leu
385                 390                 395                 400

Asp Phe Ala Leu Gln Glu Leu Leu Arg Phe Leu Arg Asn Ala Val Ser
                405                 410                 415

Ser Val Phe Leu Ser Thr Met Glu Asp Gly Arg Ile Val Lys Gly Leu
            420                 425                 430

Ala Gly Val Pro Asp Glu Gly Pro Val Leu Leu Val Gly Tyr His Met
        435                 440                 445

Leu Met Gly Leu Glu Leu Gly Pro Met Ser Glu Ala Phe Ile Lys Glu
    450                 455                 460

Lys Asn Ile Leu Phe Arg Gly Met Ala His Pro Ile Leu Tyr Ala Glu
465                 470                 475                 480

Thr Asp Ser Thr Lys Glu Phe Gly Tyr Met Asp Trp Ile Lys Val Phe
                485                 490                 495

Gly Ala Tyr Pro Val Thr Ala Thr Asn Leu Phe Lys Leu Leu Ser Ser
            500                 505                 510

Lys Ser His Val Leu Leu Tyr Pro Gly Gly Ala Arg Glu Ala Leu His
        515                 520                 525

Asn Arg Gly Glu Gln Tyr Lys Leu Ile Trp Pro Asp Gln Gln Glu Phe
    530                 535                 540

Val Arg Met Ala Ala Arg Phe Gly Ala Thr Ile Val Pro Phe Gly Thr
545                 550                 555                 560

Val Gly Glu Asp Asp Ile Ala Glu Leu Val Leu Asp Tyr Asn Asp Leu
                565                 570                 575

Met Lys Ile Pro Phe Leu Ser Asn Tyr Ile Thr Glu Ala Thr Arg Asp
            580                 585                 590

Thr Lys Glu Phe Lys Leu Arg Asp Glu Ser Ser Gly Glu Val Ala Asn
        595                 600                 605

Gln Gln Leu Tyr Leu Pro Gly Val Leu Pro Lys Ile Pro Gly Arg Phe
    610                 615                 620

Tyr Tyr Leu Phe Gly Lys Pro Ile His Thr Lys Gly Arg Pro Glu Leu
625                 630                 635                 640

Val Lys Asp Lys Glu Ala Ala Lys Leu Val Tyr Leu Glu Ala Lys Glu
                645                 650                 655

Glu Val Glu Lys Cys Ile Ala Tyr Leu Leu Lys Lys Arg Glu Glu Asp
            660                 665                 670

Pro Tyr Arg Ser Val Val Asp Arg Leu Ser Tyr Ser Leu Thr His Leu
        675                 680                 685

Pro Glu Asn Asp Val Pro Ser Phe Glu Pro
    690                 695

<210> 14
<211> 706
<212> PRT
<213> Populus trichocarpa

<400> 14

Met Thr Ser Val Val Ser Ser Gln Ala Met Tyr Cys Phe Phe Ser Thr
1               5                   10                  15

Pro Glu Ile Thr Pro Arg Phe Arg Val Arg Val Gln Ser Gly Ser Gly
            20                  25                  30

Gly Gly Asn Ser Lys Val Ser Pro Ser Asp Ser Ala Leu Val Ile Gly
        35                  40                  45

Glu Lys Asp Lys Lys Phe Gly Gly Arg Ile Asp Ser Trp Asn Ala Ser
    50                  55                  60

Leu Lys Cys Gly Ile Lys Lys Lys Ser Val Lys Asp Val Ile Ser Ser
65                  70                  75                  80

Asp Leu Asp Val Leu Trp Asp Asp Gly Tyr Gly Thr Lys Thr Ala Lys
                85                  90                  95

Asp Phe Phe Glu Gly Ala Lys Glu Met Ile Arg Pro Asp Gly Gly Pro

            100                 105                 110

Pro Arg Trp Phe Cys Pro Thr Glu Cys Gly Gln Pro Leu Lys Asp Ser
        115                 120                 125

Pro Ile Leu Leu Phe Cys Pro Gly Ile Val Gly Val Gly Leu Ala Leu
    130                 135                 140

Thr Leu His His Lys Ala Leu Gly Lys Val Phe Glu Val Arg Cys Leu
145                 150                 155                 160

His Ile Pro Val Asn Asp Arg Thr Pro Phe Glu Gly Leu Val Lys Phe
                165                 170                 175

Val Glu Glu Thr Val Arg Leu Glu His Ala Ser Ser Pro Asn Lys Pro
            180                 185                 190

Ile Tyr Leu Val Gly Asp Ser Phe Gly Gly Cys Leu Val Leu Ala Val
        195                 200                 205

Ala Ala Arg Asn Pro Glu Ile Asp Leu Val Val Ile Leu Ala Asn Pro
    210                 215                 220

Ala Thr Ser Phe Asp Arg Ser Gln Leu Arg Pro Leu Ile Pro Leu Trp
225                 230                 235                 240

Glu Ala Leu Pro Asp Gly Leu Tyr Asn Ala Leu Pro Tyr Leu Leu Ser
                245                 250                 255

Phe Val Met Gly Asn Pro Val Glu Met Ala Arg Val Asn Ile Glu Tyr
            260                 265                 270

Arg Leu Pro Pro Arg Leu Gln Ile Lys Gln Leu Phe Gln Asn Leu Ile
        275                 280                 285

Ala Leu Leu Pro His Leu Ser Asp Leu Val Asp Ile Ile Pro Lys Asp
    290                 295                 300

Thr Leu Ile Trp Lys Leu Lys Leu Leu Lys Ser Ala Ala Ser Tyr Ala
305                 310                 315                 320

Asn Ser Arg Leu His Ala Val Lys Ala Glu Val Leu Val Leu Phe Ser
                325                 330                 335

Gly Asn Asp His Met Leu Pro Ser Gly Asp Glu Ala Gln Arg Leu Lys
            340                 345                 350

Arg Thr Leu Lys Asn Cys Thr Val Arg Tyr Phe Lys Asp Asn Gly His
        355                 360                 365

Asn Ile Leu Met Glu Gly Gly Val Asn Phe Leu Ala Val Ile Lys Gly
    370                 375                 380

Thr Gly Lys Tyr Arg Arg Ser Arg Arg Ile Asp Leu Val Leu Asp Phe
385                 390                 395                 400

Ile Pro Pro Ser Met Ser Glu Phe Lys Gln Gly Tyr Asp Glu Val Phe
                405                 410                 415

Gly Leu Leu Arg Phe Ala Thr Gly Ser Ala Met Phe Ser Thr Leu Asn
            420                 425                 430

Asp Gly Lys Ile Val Lys Gly Leu His Gly Val Pro Asn Glu Gly Pro

        435                 440                 445

Val Leu Leu Val Gly Asn His Met Leu Met Gly Leu Glu Ile Tyr Ser
    450                 455                 460

Leu Val Pro Glu Phe Leu Arg Glu Lys Asn Ile Met Val Arg Val Val
465                 470                 475                 480

Val His Pro Val Val Phe Arg Glu Arg Gln Gly Val Ser Ser Pro Glu
                485                 490                 495

Phe Ser Leu Ala Asp Trp Met Lys Val Met Gly Ala Val Pro Val Thr
            500                 505                 510

Ala Ser Asn Leu Phe Asn Leu Leu Ser Thr Lys Ser His Val Leu Leu
        515                 520                 525

Tyr Pro Gly Gly Ala Arg Glu Ala Leu His Asn Arg Gly Glu Glu Tyr
    530                 535                 540

Lys Leu Phe Trp Pro Asp Gln Gln Glu Phe Val Arg Met Ala Ala Arg
545                 550                 555                 560

Phe Gly Ala Thr Ile Val Pro Phe Gly Thr Val Gly Glu Asp Asp Val
                565                 570                 575

Ala Glu Leu Val Leu Asp Tyr Asn Asp Phe Met Lys Ile Pro Val Val
            580                 585                 590

Asn Asp Tyr Ile Arg Asp Ala Asn Arg Asn Ser Ile Lys Leu Arg Asp
        595                 600                 605

Lys Ser Lys Gly Glu Val Ala Asn Gln Glu Leu Tyr Leu Pro Gly Ile
    610                 615                 620

Leu Pro Lys Val Pro Gly Arg Phe Tyr Phe Leu Phe Gly Lys Pro Ile
625                 630                 635                 640

Glu Thr Lys Asp Arg Lys Glu Glu Ile Leu Glu Asp Arg Glu Asn Ala
                645                 650                 655

Asn Gln Leu Tyr Leu His Ile Lys Ser Glu Val Glu Arg Cys Ile Ala
            660                 665                 670

Tyr Leu Leu Lys Lys Arg Glu Glu Asp Pro Tyr Arg Ser Ile Val Asp
        675                 680                 685

Arg Thr Val Tyr Arg Ala Leu His Ser Pro Leu His Glu Val Pro Ala
    690                 695                 700

Phe Asp
705

<210>  15
<211>  707
<212>  PRT
<213>  Populus trichocarpa

<400>  15

Met Thr Ser Val Val Ser Ser Gln Ala Met Tyr Cys Phe Phe Ser Thr
1               5                   10                  15

Pro Glu Ile Thr Pro Arg Phe Arg Val Arg Val Gln Ser Gly Ser Gly
            20                  25                  30

Gly Gly Asn Ser Lys Val Ser Pro Ser Asp Ser Ala Leu Val Ile Gly
        35                  40                  45

Glu Lys Asp Lys Lys Phe Gly Gly Arg Ile Asp Ser Trp Asn Ala Ser
    50                  55                  60

Leu Lys Cys Gly Ile Lys Lys Lys Ser Val Lys Asp Val Ile Ser Ser
65                  70                  75                  80

Asp Leu Asp Val Leu Trp Asp Asp Gly Tyr Gly Thr Lys Thr Ala Lys
                85                  90                  95

Asp Phe Phe Glu Gly Ala Lys Glu Met Ile Arg Pro Asp Gly Gly Pro
            100                 105                 110

Pro Arg Trp Phe Cys Pro Thr Glu Cys Gly Gln Pro Leu Lys Asp Ser
        115                 120                 125

Pro Ile Leu Leu Phe Cys Pro Gly Ile Val Gly Val Gly Leu Ala Leu
    130                 135                 140

Thr Leu His His Lys Ala Leu Gly Lys Val Phe Glu Val Arg Cys Leu
145                 150                 155                 160

His Ile Pro Val Asn Asp Arg Thr Pro Phe Glu Gly Leu Val Lys Phe
                165                 170                 175

Val Glu Glu Thr Val Arg Leu Glu His Ala Ser Ser Pro Asn Lys Pro
            180                 185                 190

Ile Tyr Leu Val Gly Asp Ser Phe Gly Gly Cys Leu Val Leu Ala Val
        195                 200                 205

Ala Ala Arg Asn Pro Glu Ile Asp Leu Val Val Ile Leu Ala Asn Pro
    210                 215                 220

Ala Thr Ser Phe Asp Arg Ser Gln Leu Arg Pro Leu Ile Pro Leu Trp
225                 230                 235                 240

Glu Ala Leu Pro Asp Gly Leu Tyr Asn Ala Leu Pro Tyr Leu Leu Ser
                245                 250                 255

Phe Val Met Gly Asn Pro Val Glu Met Ala Arg Val Asn Ile Glu Tyr
            260                 265                 270

Arg Leu Pro Pro Arg Leu Gln Ile Glu Gln Leu Phe Gln Asn Leu Ile
        275                 280                 285

Ala Leu Leu Pro His Leu Ser Asp Leu Val Asp Ile Ile Pro Lys Asp
    290                 295                 300

Thr Leu Ile Trp Lys Leu Lys Leu Leu Lys Ser Ala Ala Ser Tyr Ala
305                 310                 315                 320

Asn Ser Arg Leu His Ala Val Lys Ala Glu Val Leu Val Leu Phe Ser
                325                 330                 335

Gly Asn Asp His Met Leu Pro Ser Gly Asp Glu Ala Gln Arg Leu Lys
            340                 345                 350

Arg Thr Leu Lys Asn Cys Thr Val Arg Tyr Phe Lys Asp Asn Gly His
        355                 360                 365

Asn Ile Leu Met Glu Gly Gly Val Asn Leu Leu Thr Val Ile Lys Gly
    370                 375                 380

Thr Gly Lys Tyr Arg Arg Ser Arg Arg Ile Asp Leu Val Leu Asp Phe
385                 390                 395                 400

Ile Pro Pro Ser Met Ser Glu Phe Lys Gln Gly Tyr Asp Glu Val Ile
                405                 410                 415

Gly Leu Leu Arg Phe Ala Thr Gly Ser Ala Met Phe Ser Thr Leu Asn
            420                 425                 430

Asp Gly Lys Ile Val Lys Gly Leu His Gly Val Pro Asn Glu Gly Pro
        435                 440                 445

Val Leu Leu Val Gly Tyr His Met Leu Met Gly Leu Glu Val Tyr Ser
    450                 455                 460

Leu Val Pro Glu Phe Leu Arg Glu Lys Asn Ile Met Val Arg Gly Val
465                 470                 475                 480

Thr His Pro Val Val Phe Arg Glu Arg Gln Gly Val Ser Ser Pro Glu
                485                 490                 495

Phe Ser Leu Ala Asp Trp Met Lys Val Met Gly Ala Val Pro Val Thr
            500                 505                 510

Ala Ser Asn Leu Phe Asn Leu Leu Ser Ala Lys Ser His Val Leu Leu
        515                 520                 525

Tyr Pro Gly Gly Ala Arg Glu Ala Leu His Asn Arg Gly Glu Glu Tyr
    530                 535                 540

Lys Leu Phe Trp Pro Asp Gln Gln Glu Phe Val Arg Met Ala Ala Arg
545                 550                 555                 560

Phe Gly Ala Thr Ile Val Pro Phe Gly Thr Val Gly Glu Asp Asp Val
                565                 570                 575

Ala Glu Leu Val Leu Asp Tyr Asn Asp Phe Met Lys Ile Pro Val Ile
            580                 585                 590

Asn Asp Tyr Ile Arg Asp Ala Asn Arg Asn Ser Ile Arg Leu Arg Asp
        595                 600                 605

Lys Ser Lys Gly Glu Val Ala Asn Gln Glu Leu Tyr Leu Pro Gly Ile
    610                 615                 620

Leu Pro Lys Val Pro Gly Arg Phe Tyr Phe Leu Phe Gly Lys Pro Ile
625                 630                 635                 640

Glu Thr Lys Asp Arg Lys Glu Glu Ile Leu Glu Asp Arg Glu Asn Ala
                645                 650                 655

Asn Gln Leu Tyr Leu His Ile Lys Ser Glu Val Glu Arg Cys Ile Ala
            660                 665                 670

Tyr Leu Leu Lys Lys Arg Glu Glu Asp Pro Tyr Arg Ser Ile Val Asp
        675                 680                 685

Arg Thr Val Tyr Arg Ala Leu His Ser Pro Leu His Glu Val Pro Ala
    690                 695                 700

Phe Asp Pro
705

<210> 16
<211> 723
<212> PRT
<213> Populus trichocarpa

<400> 16

Met Ala Ser Ile Phe Thr Cys Asn Ile Ser Pro Tyr Val Ala Leu Asn
1               5                   10                  15

Ser Gly Asn Lys Pro Arg Phe Gly Val Arg Ala Gln Ser Leu Gly Gly
            20                  25                  30

Gly Asp Ser Thr Val Leu Ser Ser Asp Glu Ile Val Val Asn Gly Thr
        35                  40                  45

Ser Phe Val Gly Pro Lys Glu Lys Asn Gly Ala Leu Ile Asp Gly Gly
    50                  55                  60

Ser Gly Glu Lys Val Lys Asn Gly Thr Leu Ile Asp Gly Gly Lys Asn
65                  70                  75                  80

Gly Arg Leu Lys Pro Thr Ile Glu Lys Lys Trp Val Lys Asp Ala Val
                85                  90                  95

Phe Lys Asp Leu Glu Leu Leu Trp Asp Asp Gly His Gly Thr Lys Thr
            100                 105                 110

Val Lys Asp Tyr Leu Asp Gly Ala Lys Glu Ile Ile Lys Pro Asp Gly

        115                 120                 125

Gly Pro Pro Arg Trp Phe Cys Pro Ala Glu Cys Gly Lys Pro Leu Lys
    130                 135                 140

Asp Ser Pro Val Leu Leu Phe Leu Pro Gly Leu Asp Gly Val Gly Leu
145                 150                 155                 160

Gly Leu Ala Leu His His Lys Ala Leu Gly Lys Ala Phe Glu Val Arg
                165                 170                 175

Cys Leu His Ile Pro Val Tyr Asp Arg Thr Thr Phe Glu Gly Leu Val
            180                 185                 190

Lys Ile Val Glu Glu Thr Val Arg Leu Glu His Ala Ser Ser Pro Asn
        195                 200                 205

Lys Pro Ile Tyr Leu Val Gly Glu Ser Phe Gly Ala Cys Leu Ala Leu
    210                 215                 220

Ala Val Ala Ala Arg Asn Pro Lys Ile Asp Leu Val Leu Ile Leu Val
225                 230                 235                 240

Asn Pro Ala Thr Ser Phe Ser Arg Ser Gln Leu Pro Leu Leu Pro Ile
                245                 250                 255

Leu Glu Ala Leu Pro Asp Gly Leu His Asp Val Phe Pro Tyr Leu Val
            260                 265                 270

Gly Phe Val Thr Gly Asn Pro Val Lys Met Ala Met Ala Asn Ile Glu
        275                 280                 285

Tyr Lys Leu Pro Pro Arg Leu Gln Phe Gln Gln Leu Tyr His Asn Leu
    290                 295                 300

Thr Ala Leu Leu Pro Ser Val Ser Val Leu Ser Asp Ile Ile Pro Lys
305                 310                 315                 320

Glu Thr Leu Ile Trp Arg Leu Lys Leu Leu Lys Ser Ala Ala Ala Tyr
                325                 330                 335

Ala Asn Ser Arg Leu His Ala Val Lys Ala Glu Val Leu Val Leu Ala
            340                 345                 350

Ser Gly Asn Asp Asn Leu Leu Pro Ser Lys Asp Glu Ala His Arg Leu
        355                 360                 365

Lys Ser Ser Leu Lys Asn Cys Lys Val Arg Phe Phe Lys Asp Asn Gly
    370                 375                 380

His Ser Met Leu Met Glu Asp Gly Leu Asn Leu Leu Thr Ile Ile Lys
385                 390                 395                 400

Gly Thr Cys Lys Tyr Arg Arg Ser Arg Arg Leu Asp Leu Val Ser Asp
                405                 410                 415

Phe Leu Pro Pro Ser Met Ser Glu Phe Lys Cys Ala Phe Asp Glu Val
            420                 425                 430

Thr Gly Leu Phe Arg Leu Ala Thr Cys Ser Ala Val Phe Ser Thr Leu
        435                 440                 445

Asp Asp Gly Lys Ile Val Lys Gly Leu Ala Gly Val Pro Asp Glu Gly

450                 455                 460

Pro Val Leu Phe Ile Gly Tyr His Met Leu Met Gly Leu Glu Ile Tyr
465                 470                 475                 480

Ser Leu Val Asp Glu Phe Leu Arg Glu Lys Asn Ile Met Val Arg Gly
                485                 490                 495

Val Ala His Pro Asp Leu Phe Ser Glu Ile Met Glu Gly Ser Ser Thr
            500                 505                 510

Glu Phe Ser Val Ser Asp Trp Met Lys Val Met Gly Ala Val Pro Val
        515                 520                 525

Thr Gly Ser Asn Leu Phe Lys Leu Leu Ser Lys Lys Ser His Val Leu
    530                 535                 540

Leu Tyr Pro Gly Gly Gln Arg Glu Ala Leu His Tyr Lys Gly Glu Ala
545                 550                 555                 560

Tyr Lys Leu Ile Trp Pro Asp Gln Pro Glu Phe Val Arg Met Ala Ala
                565                 570                 575

Arg Phe Gly Ala Thr Ile Val Pro Phe Gly Thr Val Gly Glu Asp Asp
            580                 585                 590

Ile Ala Glu Leu Ala Leu Asp Tyr His Asp Leu Met Lys Ile Pro Ile
        595                 600                 605

Leu Asn Asp Phe Ala Arg Asp Leu Met Ser Lys Ser Ser Arg Val Arg
    610                 615                 620

Asp Glu Ser Lys Gly Glu Val Ala Ser Thr Asp Leu Phe Ile Pro Gly
625                 630                 635                 640

Leu Leu Pro Lys Ile Pro Gly Arg Phe Tyr Phe Leu Phe Gly Lys Pro
                645                 650                 655

Ile Lys Thr Lys Gly Met Lys Glu Met Leu Glu Asp Lys Glu Asn Ala
            660                 665                 670

Lys Gln Leu Tyr Leu His Val Lys Ser Glu Val Gln Asn Ser Ile Ala
        675                 680                 685

Tyr Leu Leu Lys Lys Arg Glu Glu Asp Pro Tyr Arg Ser Ile Ile Asp
    690                 695                 700

Arg Thr Ile Tyr Arg Ala Phe Tyr Ser Pro Leu Pro Glu Val Pro Ala
705                 710                 715                 720

Phe Asp Pro

<210>  17
<211>  682
<212>  PRT
<213>  Populus trichocarpa

<400>  17

Met Ala Ser Phe Val Thr Ser Gly Ile Leu Pro Cys Phe Gly Leu Asn
1               5                   10                  15

Ser Asp Val Lys Pro Arg Phe Gln Ala Arg Val Gln Ser Ser Ala Ser
            20                  25                  30

Val Ala Leu Lys Leu Gly Arg Leu Lys Pro Ser Ile Glu Glu Lys Lys
        35                  40                  45

Trp Val Asn Asp Phe Gly Pro Lys Asp Leu Glu Pro Leu Trp Asp Asp
    50                  55                  60

Gly Tyr Gly Thr Lys Thr Ile Lys Asp Tyr Phe Asp Gly Ala Lys Glu
65                  70                  75                  80

Met Ile Arg Pro Asp Gly Gly Pro Pro Arg Trp Phe Cys Pro Ile Glu
                85                  90                  95

Cys Gly Gln Pro Leu Lys Ser Ser Pro Thr Leu Leu Phe Leu Pro Gly
            100                 105                 110

Val Asp Gly Val Gly Leu Gly Leu Thr Leu His His Lys Ala Leu Gly
        115                 120                 125

Lys Ala Phe Glu Val Leu Cys Leu His Ile Pro Val Tyr Asp Arg Thr
    130                 135                 140

Ser Phe Glu Gly Leu Val Lys Asn Val Glu Glu Thr Val Arg Leu Glu
145                 150                 155                 160

His Ala Arg Asn Pro Asn Lys Pro Ile Tyr Leu Val Gly Glu Ser Ile
                165                 170                 175

Gly Gly Cys Leu Ala Ile Ala Val Ala Ala Arg Asn Pro Lys Val Asp
            180                 185                 190

Leu Val Val Ile Leu Ala Asn Pro Ala Thr Ser Phe Gly Arg Ser Lys
        195                 200                 205

Leu Gln Pro Phe Leu Pro Ile Leu Glu Ala Val Pro Asn Gln Leu His
    210                 215                 220

Asn Gly Phe Leu Cys Leu Leu Ser Leu Leu Thr Gly Asn Pro Val Lys
225                 230                 235                 240

Met Ala Met Val Gly Val Glu Asp Arg Leu Pro Ser Arg Leu Lys Ile
                245                 250                 255

Gly Lys Leu Tyr Gln Asn Leu Met Ala Leu Leu His Asn Leu Ser Val
            260                 265                 270

Val Ala Asp Ile Ile Pro Lys Asp Thr Leu Val Trp Lys Met Lys Leu
        275                 280                 285

Leu Arg Ser Ala Ala Asp Tyr Ala Asn Ser Cys Leu His Asn Val Lys
    290                 295                 300

Ala Glu Val Leu Leu Leu Ala Ser Glu Lys Asp Arg Leu Leu Pro Ser
305                 310                 315                 320

Arg Asp Glu Ala Leu Arg Leu Lys Ser Leu Leu Gln Asn Cys Thr Val
                325                 330                 335

Arg Asn Phe Lys Ala Asn Gly His Ala Ile Leu Leu Glu Asp Gly Val
            340                 345                 350

Gly Leu Leu Thr Ala Ile Lys Gly Thr Ser Lys Tyr Arg Arg Ser Lys
        355                 360                 365

Arg Ile Asp Phe Val Ser Asp Tyr Leu Pro Pro Ser Thr Ser Glu Phe
    370                 375                 380

Lys Ser Phe Phe Glu Glu Ala Tyr Gly Leu Leu Leu Tyr Ala Ala Gly
385                 390                 395                 400

Ser Thr Met Phe Ser Thr Leu Glu Asp Gly Lys Ile Val Lys Gly Leu
                405                 410                 415

Ala Gly Val Pro Asn Glu Gly Pro Val Leu Leu Val Gly Tyr His Met
            420                 425                 430

Leu Met Ala Phe Asp Ile Tyr Pro Leu Gly Glu Gly Phe Leu Arg Glu
        435                 440                 445

Lys Asn Ile Met Val Arg Gly Leu Gly His Pro Asp Leu Phe Thr Gly
    450                 455                 460

Lys Leu Glu Asp Ser Ser Asn Glu Phe Ala Tyr Ala Glu Trp Ile Arg
465                 470                 475                 480

Val Met Gly Thr Val Ala Gly Thr Ala Ser Asn Leu Phe Lys Leu Leu
                485                 490                 495

Ser Thr Lys Ser His Val Val Leu Tyr Pro Gly Gly Ala Arg Glu Ser
            500                 505                 510

Leu His Asn Lys Gly Glu Glu Tyr Lys Leu Phe Trp Pro Asp Gln Gln
        515                 520                 525

Glu Phe Val Arg Thr Ala Ala Arg Phe Gly Ala Thr Ile Val Pro Phe
    530                 535                 540

Gly Thr Val Gly Glu Asp Asp Leu Thr His Leu Val Leu Asp Tyr His
545                 550                 555                 560

Asp Met Met Lys Ile Pro Ile Val Ser Asp Tyr Ile Arg Glu Val Asn
                565                 570                 575

Ser Lys Ala Thr Arg Ile Arg Asp Asn Ser Lys Gly Glu Val Ala Asn
            580                 585                 590

Gln Gln Val Tyr Ile Pro Gly Val Leu Pro Lys Leu Pro Gly Arg Phe
        595                 600                 605

Tyr Tyr Leu Phe Gly Lys Pro Ile Lys Thr Lys Gly Met Glu Asp Met
    610                 615                 620

Leu Arg Asp Arg Glu Asn Ala Asn Gln Leu Tyr Leu His Val Lys Ser
625                 630                 635                 640

Glu Val Glu Asn Asn Ile Ala Tyr Leu Leu Lys Lys Arg Glu Glu Asp
                645                 650                 655

Pro Tyr Arg Ser Leu Ile Asn Arg Thr Ile Tyr Gln Ala Leu His Ser
            660                 665                 670

Pro Ser Ser Asn Val Pro Thr Phe Asp Pro
        675                 680

<210>  18
<211>  35

<212>  DNA
<213>  Artificial

<220>
<223>  Synthetic DNA

<400>  18
aaaaagcagg ctcaatggcg gatacagcta gagga                                35


<210>  19
<211>  36
<212>  DNA
<213>  Artificial

<220>
<223>  Synthetic DNA

<400>  19
ctcgcccttg ctcaccatag tagtgtgctg gccacc                               36


<210>  20
<211>  36
<212>  DNA
<213>  Artificial

<220>
<223>  Synthetic DNA

<400>  20
ggtggccagc acactactat ggtgagcaag ggcgag                               36


<210>  21
<211>  34
<212>  DNA
<213>  Artificial

<220>
<223>  Synthetic DNA

<400> 21
agaaagctgg gtcttacttg tacagctcgt ccat 34

<210> 22
<211> 29
<212> DNA
<213> Artificial

<220>
<223> Synthetic DNA

<400> 22
ggggacaagt ttgtacaaaa aagcaggct 29

<210> 23
<211> 28
<212> DNA
<213> Artificial

<220>
<223> Synthetic DNA

<400> 23
ggggaccact ttgtacaaga aagctggg 28

<210> 24
<211> 1239
<212> DNA
<213> Artificial

<220>
<223> Synthetic DNA coding an Oleosin-GFP fusion gene

<220>
<221> CDS
<222> (1)..(1239)

<400> 24

atg gcg gat aca gct aga gga acc cat cac gat atc atc ggc aga gac 48
Met Ala Asp Thr Ala Arg Gly Thr His His Asp Ile Ile Gly Arg Asp
1               5                   10                  15

cag tac ccg atg atg ggc cga gac cga gac cag tac cag atg tcc gga 96
Gln Tyr Pro Met Met Gly Arg Asp Arg Asp Gln Tyr Gln Met Ser Gly
            20                  25                  30

cga gga tct gac tac tcc aag tct agg cag att gct aaa gct gca act 144
Arg Gly Ser Asp Tyr Ser Lys Ser Arg Gln Ile Ala Lys Ala Ala Thr
        35                  40                  45

gct gtc aca gct ggt ggt tcc ctc ctt gtt ctc tcc agc ctt acc ctt 192
Ala Val Thr Ala Gly Gly Ser Leu Leu Val Leu Ser Ser Leu Thr Leu
    50                  55                  60

gtt gga act gtc ata gct ttg act gtt gca aca cct ctg ctc gtt atc 240
Val Gly Thr Val Ile Ala Leu Thr Val Ala Thr Pro Leu Leu Val Ile
65                  70                  75                  80

ttc agc cca atc ctt gtc ccg gct ctc atc aca gtt gca ctc ctc atc 288
Phe Ser Pro Ile Leu Val Pro Ala Leu Ile Thr Val Ala Leu Leu Ile
                85                  90                  95

acc ggt ttt ctt tcc tct gga ggg ttt ggc att gcc gct ata acc gtt 336
Thr Gly Phe Leu Ser Ser Gly Gly Phe Gly Ile Ala Ala Ile Thr Val
            100                 105                 110

ttc tct tgg att tac aag tac gca acg gga gag cac cca cag gga tca 384
Phe Ser Trp Ile Tyr Lys Tyr Ala Thr Gly Glu His Pro Gln Gly Ser
        115                 120                 125

gac aag ttg gac agt gca agg atg aag ttg gga agc aaa gct cag gat 432
Asp Lys Leu Asp Ser Ala Arg Met Lys Leu Gly Ser Lys Ala Gln Asp
    130                 135                 140

ctg aaa gac aga gct cag tac tac gga cag caa cat act ggt ggg gaa 480
Leu Lys Asp Arg Ala Gln Tyr Tyr Gly Gln Gln His Thr Gly Gly Glu
145                 150                 155                 160

cat gac cgt gac cgt act cgt ggt ggc cag cac act act atg gtg agc 528
His Asp Arg Asp Arg Thr Arg Gly Gly Gln His Thr Thr Met Val Ser

                165                 170                 175

aag ggc gag gag ctg ttc acc ggg gtg gtg ccc atc ctg gtc gag ctg 576
Lys Gly Glu Glu Leu Phe Thr Gly Val Val Pro Ile Leu Val Glu Leu
            180                 185                 190

gac ggc gac gtg aac ggc cac aag ttc agc gtg tcc ggc gag ggc gag 624
Asp Gly Asp Val Asn Gly His Lys Phe Ser Val Ser Gly Glu Gly Glu
        195                 200                 205

ggc gat gcc acc tac ggc aag ctg acc ctg aag ttc atc tgc acc acc 672
Gly Asp Ala Thr Tyr Gly Lys Leu Thr Leu Lys Phe Ile Cys Thr Thr
    210                 215                 220

ggc aag ctg ccc gtg ccc tgg ccc acc ctc gtg acc acc ttc acc tac 720
Gly Lys Leu Pro Val Pro Trp Pro Thr Leu Val Thr Thr Phe Thr Tyr
225                 230                 235                 240

ggc gtg cag tgc ttc agc cgc tac ccc gac cac atg aag cag cac gac 768
Gly Val Gln Cys Phe Ser Arg Tyr Pro Asp His Met Lys Gln His Asp
                245                 250                 255

ttc ttc aag tcc gcc atg ccc gaa ggc tac gtc cag gag cgc acc atc 816
Phe Phe Lys Ser Ala Met Pro Glu Gly Tyr Val Gln Glu Arg Thr Ile
            260                 265                 270

ttc ttc aag gac gac ggc aac tac aag acc cgc gcc gag gtg aag ttc 864
Phe Phe Lys Asp Asp Gly Asn Tyr Lys Thr Arg Ala Glu Val Lys Phe
        275                 280                 285

gag ggc gac acc ctg gtg aac cgc atc gag ctg aag ggc atc gac ttc 912
Glu Gly Asp Thr Leu Val Asn Arg Ile Glu Leu Lys Gly Ile Asp Phe
    290                 295                 300

aag gag gac ggc aac atc ctg ggg cac aag ctg gag tac aac tac aac 960
Lys Glu Asp Gly Asn Ile Leu Gly His Lys Leu Glu Tyr Asn Tyr Asn
305                 310                 315                 320

agc cac aac gtc tat atc atg gcc gac aag cag aag aac ggc atc aag 1008
Ser His Asn Val Tyr Ile Met Ala Asp Lys Gln Lys Asn Gly Ile Lys
                325                 330                 335

gtg aac ttc aag atc cgc cac aac atc gag gac ggc agc gtg cag ctc     1056
Val Asn Phe Lys Ile Arg His Asn Ile Glu Asp Gly Ser Val Gln Leu
            340                 345                 350

gcc gac cac tac cag cag aac acc ccc atc ggc gac ggc ccc gtg ctg     1104
Ala Asp His Tyr Gln Gln Asn Thr Pro Ile Gly Asp Gly Pro Val Leu
        355                 360                 365

ctg ccc gac aac cac tac ctg agc acc cag tcc gcc ctg agc aaa gac     1152
Leu Pro Asp Asn His Tyr Leu Ser Thr Gln Ser Ala Leu Ser Lys Asp
    370                 375                 380

ccc aac gag aag cgc gat cac atg gtc ctg ctg gag ttc gtg acc gcc     1200
Pro Asn Glu Lys Arg Asp His Met Val Leu Leu Glu Phe Val Thr Ala
385                 390                 395                 400

gcc ggg atc act cac ggc atg gac gag ctg tac aag taa                 1239
Ala Gly Ile Thr His Gly Met Asp Glu Leu Tyr Lys
                405                 410

<210>  25
<211>  412
<212>  PRT
<213>  Artificial

<220>
<223>  Synthetic Construct

<400>  25

Met Ala Asp Thr Ala Arg Gly Thr His His Asp Ile Ile Gly Arg Asp
1               5                   10                  15

Gln Tyr Pro Met Met Gly Arg Asp Arg Asp Gln Tyr Gln Met Ser Gly
            20                  25                  30

Arg Gly Ser Asp Tyr Ser Lys Ser Arg Gln Ile Ala Lys Ala Ala Thr
        35                  40                  45

Ala Val Thr Ala Gly Gly Ser Leu Leu Val Leu Ser Ser Leu Thr Leu
    50                  55                  60

Val Gly Thr Val Ile Ala Leu Thr Val Ala Thr Pro Leu Leu Val Ile
65                  70                  75                  80

Phe Ser Pro Ile Leu Val Pro Ala Leu Ile Thr Val Ala Leu Leu Ile
                85                  90                  95

Thr Gly Phe Leu Ser Ser Gly Gly Phe Gly Ile Ala Ala Ile Thr Val
            100                 105                 110

Phe Ser Trp Ile Tyr Lys Tyr Ala Thr Gly Glu His Pro Gln Gly Ser
        115                 120                 125

Asp Lys Leu Asp Ser Ala Arg Met Lys Leu Gly Ser Lys Ala Gln Asp
    130                 135                 140

Leu Lys Asp Arg Ala Gln Tyr Tyr Gly Gln Gln His Thr Gly Gly Glu
145                 150                 155                 160

His Asp Arg Asp Arg Thr Arg Gly Gly Gln His Thr Thr Met Val Ser
                165                 170                 175

Lys Gly Glu Glu Leu Phe Thr Gly Val Val Pro Ile Leu Val Glu Leu
            180                 185                 190

Asp Gly Asp Val Asn Gly His Lys Phe Ser Val Ser Gly Glu Gly Glu
        195                 200                 205

Gly Asp Ala Thr Tyr Gly Lys Leu Thr Leu Lys Phe Ile Cys Thr Thr

210                 215                 220

Gly Lys Leu Pro Val Pro Trp Pro Thr Leu Val Thr Thr Phe Thr Tyr
225                 230                 235                 240

Gly Val Gln Cys Phe Ser Arg Tyr Pro Asp His Met Lys Gln His Asp
                245                 250                 255

Phe Phe Lys Ser Ala Met Pro Glu Gly Tyr Val Gln Glu Arg Thr Ile
            260                 265                 270

Phe Phe Lys Asp Asp Gly Asn Tyr Lys Thr Arg Ala Glu Val Lys Phe
        275                 280                 285

Glu Gly Asp Thr Leu Val Asn Arg Ile Glu Leu Lys Gly Ile Asp Phe
    290                 295                 300

Lys Glu Asp Gly Asn Ile Leu Gly His Lys Leu Glu Tyr Asn Tyr Asn
305                 310                 315                 320

Ser His Asn Val Tyr Ile Met Ala Asp Lys Gln Lys Asn Gly Ile Lys
                325                 330                 335

Val Asn Phe Lys Ile Arg His Asn Ile Glu Asp Gly Ser Val Gln Leu
            340                 345                 350

Ala Asp His Tyr Gln Gln Asn Thr Pro Ile Gly Asp Gly Pro Val Leu
        355                 360                 365

Leu Pro Asp Asn His Tyr Leu Ser Thr Gln Ser Ala Leu Ser Lys Asp
    370                 375                 380

Pro Asn Glu Lys Arg Asp His Met Val Leu Leu Glu Phe Val Thr Ala
385                 390                 395                 400

Ala Gly Ile Thr His Gly Met Asp Glu Leu Tyr Lys
                405                 410

## Description

### Technical Field

The present invention relates to a mutant gene that regulates the oil-and-fat content in seeds, which has been identified using changes in oil bodies existing in seeds as an indicator, and a method for regulating the oil-and-fat content in seeds through mutation of a given gene.

### Background Art

Oil bodies (occasionally referred to as "lipid bodies") are organelles that are present in large quantities in plants, and particularly in seed cells of oil crops. Oil bodies comprise a monolayer membrane of phospholipid comprising specific proteins referred to as oleosins, steroleosins, and caleosins, and they accumulate plant oil-and-fats in the form of triacylglycerol (TAG, neutral fat, or neutral lipid) therein. In particular, large quantities of plant fats are accumulated in plant seeds. In the past, oil-and-fats accumulated in oil bodies have been analyzed by methods that involve pulverizing seeds to extract oil-and-fat components and performing gas chromatography, liquid chromatography, or other techniques. Such analytical methods, however, required the addition of lipid degradation inhibitors and processing at low temperatures. In addition, oil-and-fat components may be disadvantageously degraded.

Siloto, R. M. P. et al., Plant Cell 18, 1961-1974, 2006 discloses that oil body size is influenced by oleosin quantity. Wahlroos et al., GENESIS, 35 (2): 125-132, 2003 discloses that the oleosin gene is fused to the green fluorescent protein (GFP) gene and oil bodies can be observed with the aid of GFP fluorescence. Even if it is possible to observe oil bodies, however, the correlation between the number or form of oil bodies and the oil-and-fat amount or type accumulated in oil bodies has not yet been elucidated.

### Disclosure of the Invention

The present invention is intended to establish a system that allows measurement of various properties, such as the number or size of oil bodies in plant cells. Such system elucidates the correlation between properties of oil bodies and the oil-and-fat content. The present invention is also intended to provide a mutant gene having the function of regulating the oil-and-fat content in seeds and a method for regulating the oil-and-fat content in seeds.

The present inventors have conducted concentrated studies in order to attain the above objects, expressed an oleosin-GFP fusion protein, and discovered a positive correlation between the sum of GFP fluorescence intensities and oil-and-fat content. Based on such findings, the present inventors discovered a gene having the function of influencing the oil-and-fat content in seeds and the influence of a mutation of an amino acid substitution in such gene on the oil-and-fat content in seeds, thereby completing the present invention.

Specifically, the mutant gene of the present invention includes the following.
(1) A mutant gene encoding a mutant protein having a consensus sequence derived from the amino acid sequence as shown in SEQ ID NO: 1 by substitution of the 12th amino acid residue from the N terminus with another amino acid and having the function of regulating the oil-and-fat content in plant seeds.
(2) The mutant gene according to (1), wherein the other amino acid is an amino acid selected from the group consisting of isoleucine, valine, leucine, and methionine.
(3) The mutant gene according to (1), wherein the other amino acid is isoleucine.
(4) The mutant gene according to (1), wherein the mutant protein has a sequence derived from the consensus sequence included in an amino acid sequence as shown in any one of SEQ ID NOs: 4 to 17 by substitution of the 12th amino acid residue from the N terminus with another amino acid.
(5) The mutant gene according to (1), which is a mutant of a plant-derived gene.
(6) The mutant gene according to (1), which is a mutant of a gene derived from a plant belonging to a family selected from the group consisting of *Gramineae, Brassicaceae,* and *Salicaceae.*
(7) The mutant gene according to (1), which is a mutant of a gene derived from a plant belonging to a species selected from the group consisting of barley, rice, *Arabidopsis thaliana, Brassica rapa,* and *Populus nigra.*
   In addition, the present invention includes a mutant protein encoded by the mutant gene mentioned above, a transgenic cell into which the mutant gene has been introduced, and a transgenic plant into which the mutant gene has been introduced.
   The methods for regulating the oil-and-fat content in seeds of the present invention include the following.
(8) A method for regulating the oil-and-fat content in seeds comprising introducing a mutation in which the 12th amino acid residue from the N terminus of the amino acid sequence as shown in SEQ ID NO: 1 is substituted with another amino acid into a gene encoding a protein having a consensus sequence comprising the amino acid sequence as shown in SEQ ID NO: 1.
(9) The method for regulating the oil-and-fat content in seeds according to (8), wherein the other amino acid is an amino acid selected from the group consisting of isoleucine, valine, leucine, and methionine.
(10) The method for regulating the oil-and-fat content in seeds according to (8), wherein the other amino acid is isoleucine.
(11) The method for regulating the oil-and-fat content in seeds according to (8), wherein a mutant protein resulting from substitution of the 12th amino acid residue from the N terminus with another amino acid in the consensus sequence included in an amino acid sequence as shown in any one of SEQ ID NOs: 4 to 17 is expressed.
(12) The method for regulating the oil-and-fat content in seeds according to (8), wherein a gene derived from a plant belonging to a family selected from the group consisting of *Gramineae, Brassicaceae,* and *Salicaceae* is varied.
(13) The method for regulating the oil-and-fat content in seeds according to (8), wherein a gene derived from a plant belonging to a species selected from the group consisting of barley, rice, *Arabidopsis thaliana, Brassica rapa,* and *Populus nigra* is varied.

This description includes part or all of the contents as disclosed in the description and/or drawings of Japanese Patent Application No. 2008-048612, which is a priority document of the present application.

### Brief Description of the Drawings

Fig. 1 schematically shows the amino acid substitution scoring matrix (BLOSUM).
Fig. 2-1 is an alignment diagram showing the results of alignment analysis of 14 types of amino acid sequences.
Fig. 2-2 is an alignment diagram showing the results of alignment analysis of 14 types of amino acid sequences.

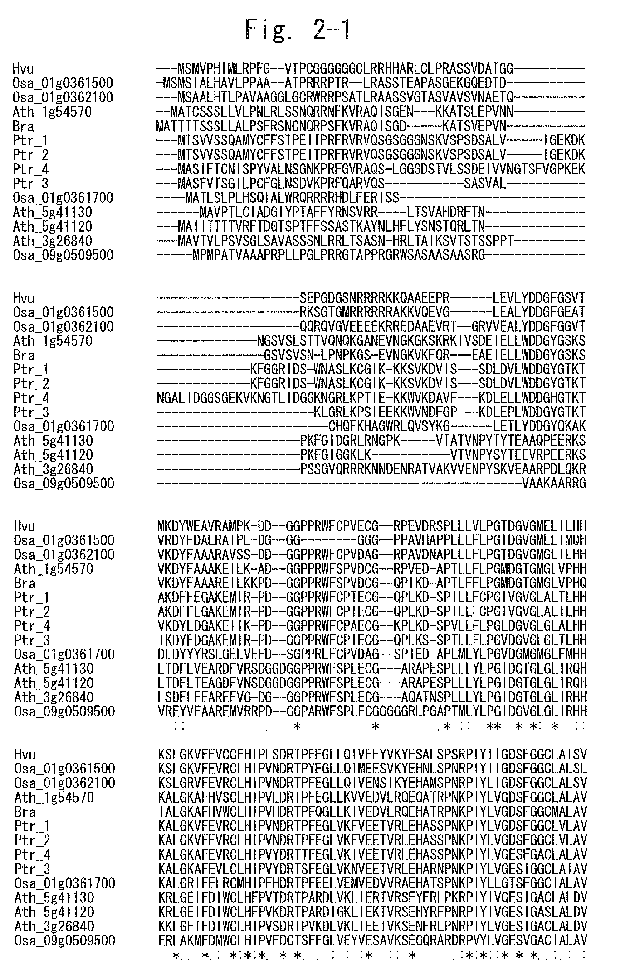

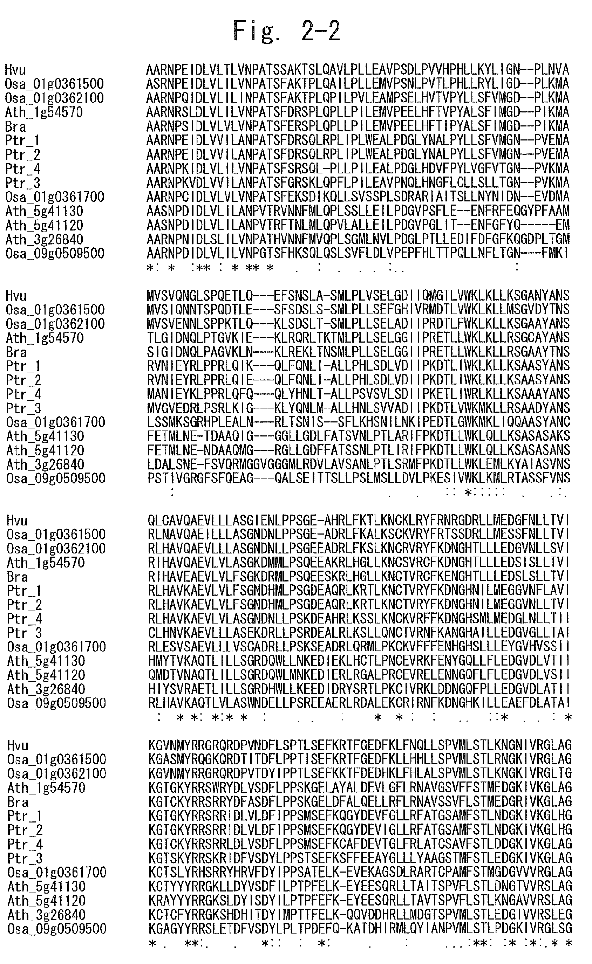

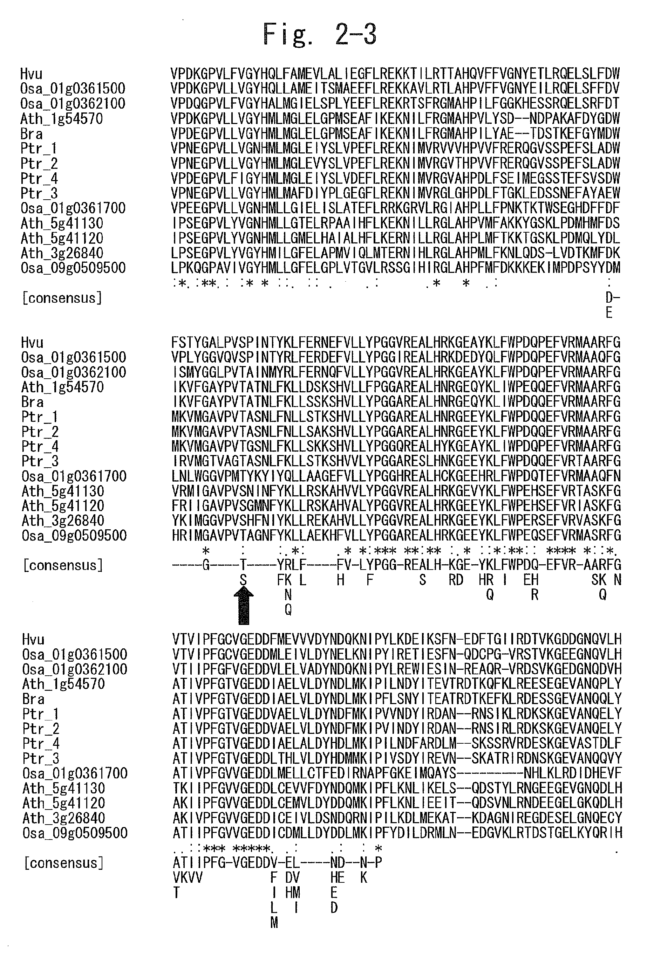

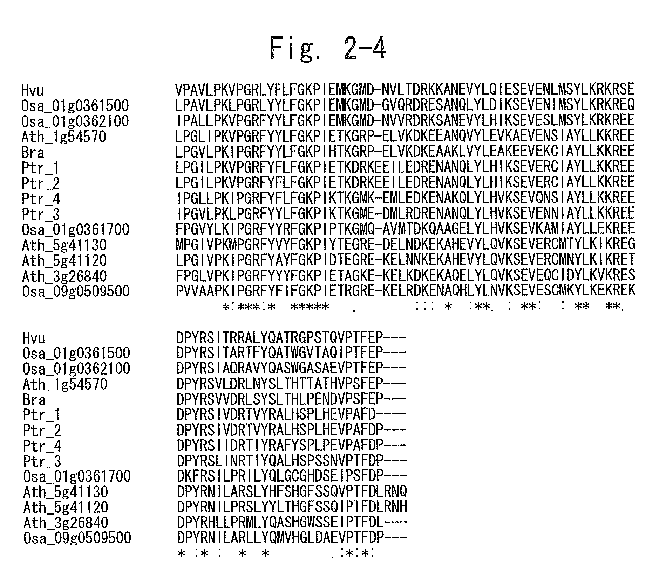

Fig. 2-3 is an alignment diagram showing the results of alignment analysis of 14 types of amino acid sequences.
Fig. 2-4 is an alignment diagram showing the results of alignment analysis of 14 types of amino acid sequences.

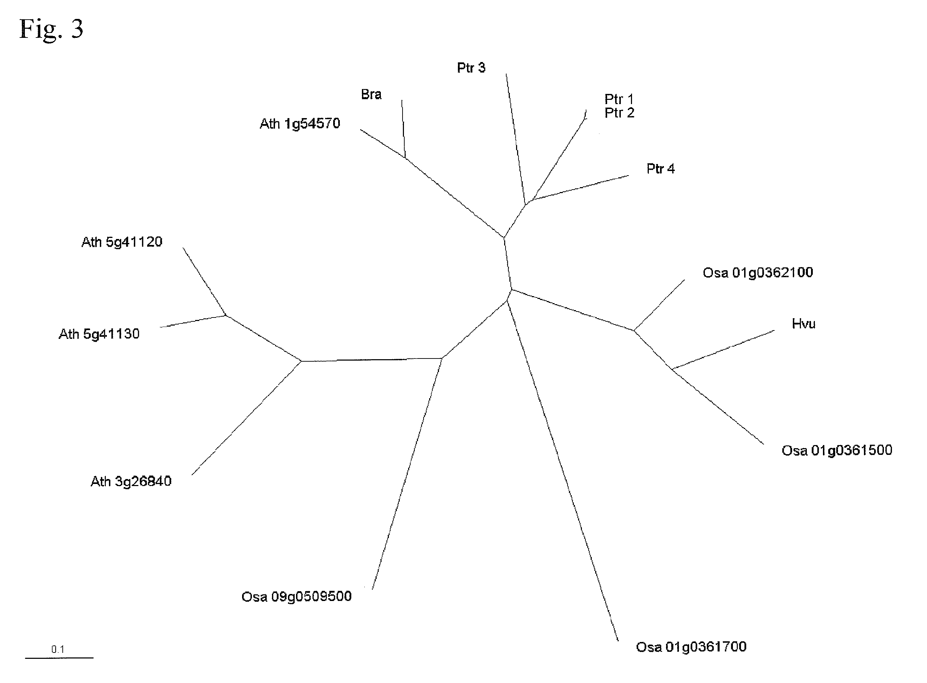

Fig. 3 shows a molecular evolution dendrogram obtained from the alignment diagram shown in Fig. 2.

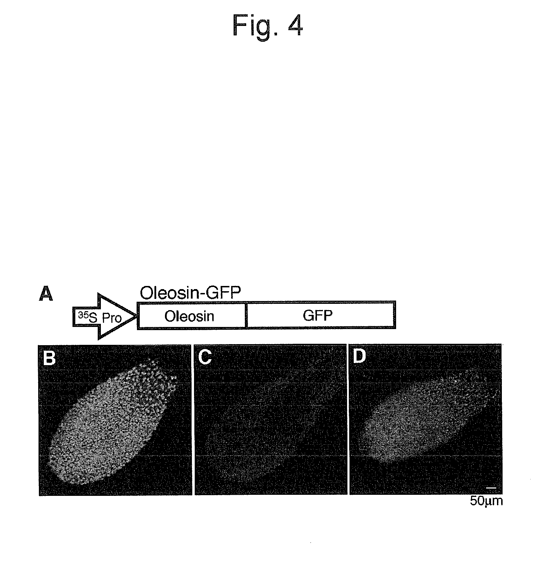

Fig. 4A schematically shows the constitution of the oleosin-GFP fusion gene, and Figs. 4B to 4D each show a fluorescence picture of cotyledons of OleG, mutant A, and mutant B 6 days after the initiation of germination in the dark.

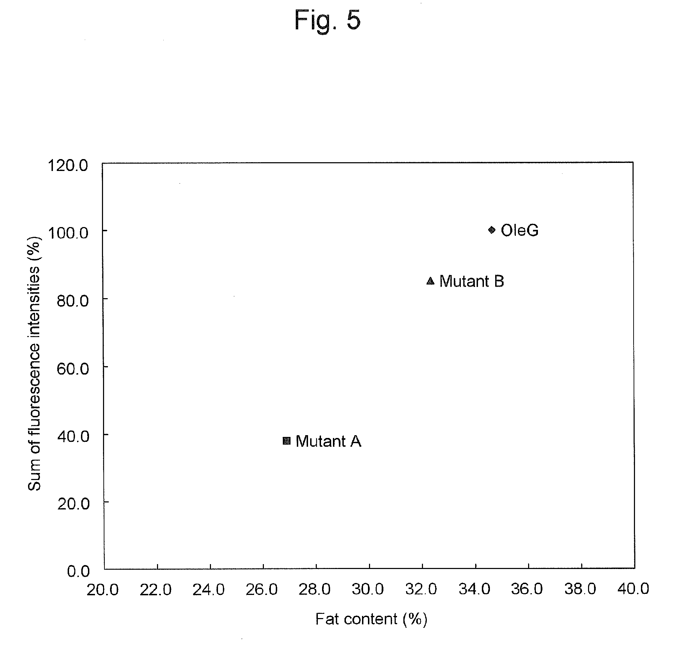

Fig. 5 is a characteristic diagram showing the correlation of the sum of GFP fluorescence (%) and the oil-and-fat content in seeds.

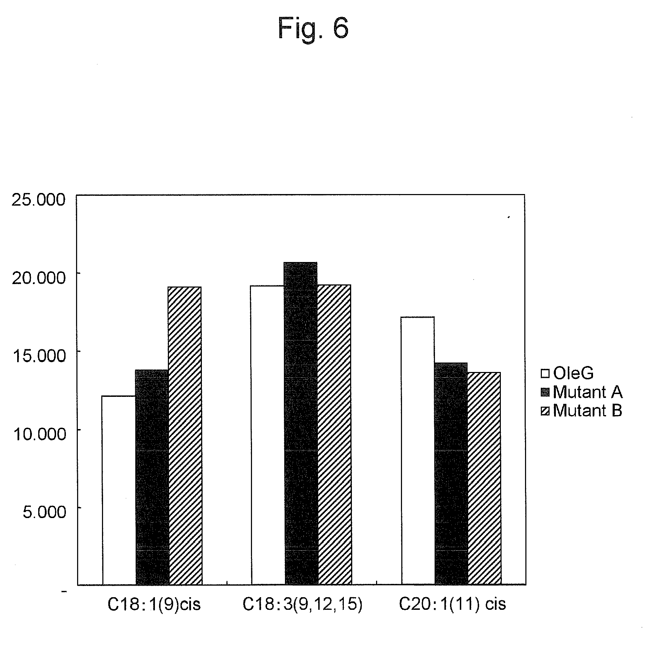

Fig. 6 is a characteristic diagram showing the results of analysis of fatty acid compositions of seed reserved lipids of OleG, mutant A, and mutant B.

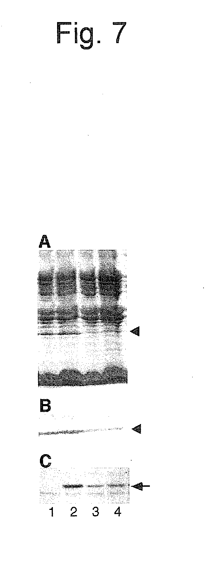

Fig. 7 is a photograph showing the results of SDS-PAGE using protein samples extracted from seeds of wild-type *Arabidopsis thaliana,* OleG, mutant A, and mutant B, the results of immunoblot analysis using an anti-oleosin antibody, and the results of immunoblot analysis using an anti-GFP antibody (C).

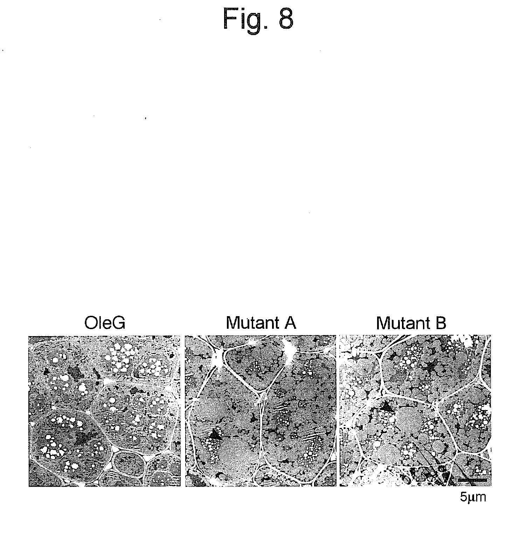

Fig. 8 is a photograph showing the results of electron microscopic observation of seed cells of OleG, mutant A, and mutant B.

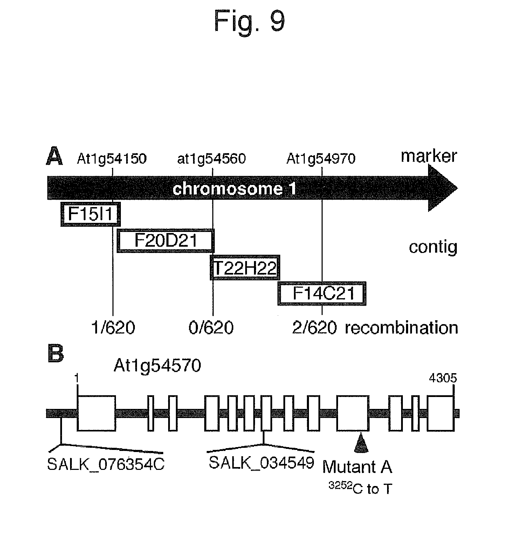

Fig. 9A is a characteristic diagram showing the results of high-precision mapping for identifying a causal gene of mutant A and Fig 9B schematically shows the structure of the gene (At1g54570) that is present in the mapped position.

### Best Modes for Carrying out the Invention

Hereafter, the present invention is described in detail with reference to the drawings.

In the present invention, an oil body-specific protein and a fluorescent protein (more particularly the oleosin-GFP fusion gene) were used to transform a model plant *Arabidopsis thaliana,* and oil bodies contained in the cotyledons obtained from the transgenic *Arabidopsis thaliana* plant were fluorescently visualized. A mutation was induced in such transgenic *Arabidopsis thaliana* plant, changes in various properties, such as the configuration and the number of oil bodies, were observed, and changes in the oil-and-fat content and oil compositions were measured. Surprisingly, a positive correlation between the fluorescence sum in cotyledons (i.e., fluorescence intensity per unit area) among various properties of oil bodies and the oil-and-fat content in seeds was found. Based on such finding, a causal gene for a mutant that changes in the oil-and-fat content, the oil-and-fat amount, and the fatty acid composition in the oil-and-fat in a plant and a causative mutation were identified.

Specifically, the mutant gene of the present invention encodes a protein resulting from substitution of a given amino acid in the evolutionarily conserved amino acid sequence referred to as the "diacylglycerol acyltransferase domain" with another amino acid and having the function of regulating the oil-and-fat content and the oil-and-fat amount in a plant. The mutant gene of the present invention is a protein having an evolutionarily conserved amino acid sequence referred to as the diacylglycerol acyltransferase domain comprising an amino acid sequence resulting from substitution of a given amino acid with another amino acid. In the description below, an amino acid sequence constituting a evolutionarily conserved region referred to as the diacylglycerol acyltransferase domain is referred to as the "consensus sequence." SEQ ID NO: 1 shows the consensus sequence.

Specifically, the consensus sequence shown in SEQ ID NO: 1 can also be represented as the amino acid sequence below (using a single-letter code for the amino acids). (D/E)xxxxxGxxxx(T/S)xxxx(Y/F)(R/K/N/Q)L(F/L)xxxx(F/H)VxL(Y/F)PGGxRE(A/S)LHx( K/R)(G/D)Ex(Y/H)(K/R/Q)L(F/I)WP(D/E)(Q/H/R)xEFVRxA(A/S)(R/K/Q)F(G/N)(A/V/T)(T /K)(I/V)(I/V)PFGxVGEDD(V/F/I/L/M)x(E/D/H)(L/V/M/I)x

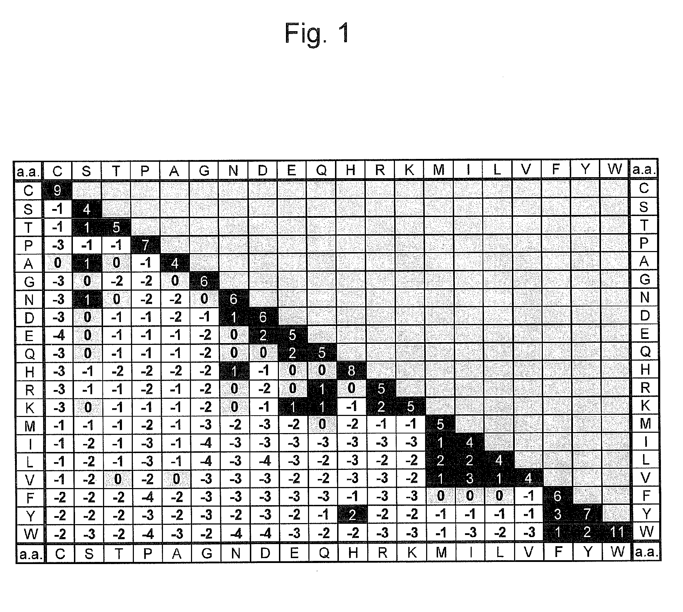

In such amino acid sequence, each plurality of amino acids in parentheses individually represents the variation of an amino acid residue in the position of interest. In the amino acid sequence, x signifies that arbitrary amino acid residues can be used at such position. Such variation of amino acid residues that can be used in a given position occurs for the following reasons. Specifically, the aforementioned consensus sequence can be normalized by subjecting the amino acid sequences of homologous proteins derived from a plurality of plant species to multiple alignment analysis. As described in Reference (1) "McKee's Biochemistry," Vol. 3, Chap. 5, Amino acid, Peptide, Protein, 5.1 Amino acid, Atsushi Ichikawa (editor), Shinichi Fukuoka (translator), Ryosuke Sone (publisher), published by Kagaku-Dojin Publishing Co., Inc., ISBN4-7598-0944-9, it is well-known that amino acids are classified in accordance with side chains having similar properties (i.e., chemical properties or physical size). Also, it is well-known that, in molecular evolution, substitution of amino acid residues, which are classified into a given group, takes place at high frequency while protein activity is retained. Based on such concept, Reference (2): Henikoff S., Henikoff J. G., Amino-acid substitution matrices from protein blocks, Proc. Natl. Acad. Sci., U.S.A., 89, 10915-10919, 1992 proposes in its Fig. 2 the amino acid substitution scoring matrix (BLOSUM), and this is extensively used (Fig. 1 of the present description). Reference (2) is based on the principle that substitution of amino acids having similar side chain chemical properties imposes little influence on proteins in terms of structural or functional changes. According to References (1) and (2), side chain groups of amino acids in terms of the multiple alignment can be determined based on indicators such as chemical properties or physical sizes. This is indicated as a group of amino acids having a score of 0 or larger, and preferably as a group of amino acids having a score of 1 or larger, as shown in Fig. 1. Examples of representative groups include the 8 groups shown below. Other more specific classifications may be a group of amino acids having values of 0 or higher, preferably a group of amino acids having values of 1 or higher, and more preferably a group of amino acids having values of 2 or higher, as shown in Fig. 1.

### 1) Group of hydrophobic aliphatic amino acids (ILMV group)

This is a group of amino acids having aliphatic hydrophobic side chains selected from among the neutral and non-polar amino acids described in Reference (1), which is composed of V (Val, valine), L (Leu, leucine), I (Ile, isoleucine), and M (Met, methionine). Among amino acids that are classified as neutral and non-polar amino acids according to Reference (1), FGACWP is not included in "the group of aliphatic hydrophobic amino acids" for the following reasons. That is, G (Gly, glycine) and A (Ala, alanine) are smaller than methyl groups and have small non-polar effects. Also, C (Cys, cysteine) occasionally plays a key role in S-S bonds and forms a hydrogen bond with an oxygen or nitrogen atom. Further, side chains of F (Phe, phenylalanine) and W (Trp, tryptophan) have very large molecular weights and have potent aromatic effects. Also, P (Pro, proline) has potent imino acid effects and disadvantageously immobilizes the angle of the polypeptide main chain.

### 2) Group having hydroxymethylene group (ST group)

This is a group of amino acids having hydroxymethylene groups on the side chains selected from among the neutral and polar amino acids, which is composed of S (Ser, serine) and T (Thr, threonine). Since hydroxyl groups that are present on S and T side chains are sugar-binding sites, such hydroxyl groups often serve as important sites allowing a given polypeptide (a protein) to have a given activity.

### 3) Group of acidic amino acids (DE group)

This is a group of amino acids having acidic carboxyl groups on the side chains, which is composed of D (Asp, aspartic acid) and E (Glu, glutamic acid).

### 4) Group of basic amino acids (KR group)

This is a group of basic amino acids, which is composed of K (Lys, lysine) and R (Arg, arginine). K and R positively charge over a wide pH range and have basic properties. H (His, histidine), which is classified as a basic amino acid, is not substantially ionized at pH 7, and thus it is not classified into this group.

### 5) Group of amino acids having methylene groups or polar groups (DHN groups)

Amino acids of this group have carbon atoms at the α positions, methylene groups bound thereto as side chains, and polar groups at farther positions. Physical sizes of non-polar methylene groups are very similar, and the group is composed of N (Asn, asparagine; an amide group as a polar group), D (Asp, aspartic acid; a carboxyl group as a polar group), and H (His, histidine; an imidazole group as a polar group).

### 6) Group of amino acids having dimethylene groups or polar groups (EKQR groups)

Amino acids of this group have carbon atoms at the α positions, linear hydrocarbons of dimethylene or higher bound thereto as side chains, and polar groups at farther positions. Physical sizes of non-polar dimethylene groups are very similar, and such groups are composed of E (Glu, glutamic acid; a carboxyl group as a polar group), K (Lys, an amino group as a polar group), Q (Gln, glutamine; an amide group as a polar group), and R (Arg, arginine; imino and amino groups as a polar groups).

### 7) Group of aromatic amino acids (FYW group)

This is a group of aromatic amino acids having benzene nuclei on the side chains, and this group has chemical properties peculiar to aromatic amino acids. The group is composed of F (Phe, phenylalanine), Y (Tyr, tyrosine), and W (Trp, tryptophan).

### 8) Group of cyclic and polar amino acids (HY group)

This group is composed of amino acids simultaneously having a cyclic structure and a polar group on the side chains. The group is composed of H (H, histidine; the cyclic structure and the polar group are imidazole groups) and Y (Tyr, tyrosine; the cyclic structure is the benzene nucleus and the polar group is a hydroxyl group).

The mutant gene of the present invention results from substitution of the 12th amino acid residue from the N terminus in the aforementioned consensus sequence (i.e., threonine (T) or serine (S)) with another amino acid, and it encodes a protein having the function of regulating the oil-and-fat amount in a plant. The 12th amino acid residue from the N terminus in the aforementioned consensus sequence (i.e., threonine (T) or serine (S)) is an amino acid residue that is highly likely to be phosphorylated by threonine kinase or serine kinase, and it is highly likely to be an amino acid residue that remarkably contributes to diacylglycerol acyltransferase activity. By substituting the 12th amino acid residue from the N terminus in the aforementioned consensus sequence (i.e., threonine (T) or serine (S)) with another amino acid, accordingly, the activity of a protein encoded by the gene of the present invention would be changed. The situation in which activity of a protein encoded by the gene of the present invention would be changed refers to a situation in which enzyme activity is improved, enzyme activity is inhibited, substrate specificity is changed, or affinity with a substrate, a coenzyme, or another factor is changed.

Examples of other amino acids after substitution include isoleucine, valine, leucine, and methionine, with isoleucine being particularly preferable. When the 12th amino acid residue from the N terminus in the aforementioned consensus sequence (i.e., threonine (T) or serine (S)) is substituted with isoleucine, the resultant exhibits a characteristic phenotype. That is, the oil-and-fat amount per seed grain is increased; however, the oil-and-fat content (%) per seed grain is decreased in comparison with that of a wild-type plant.

The consensus sequence may be the sequence (shown in SEQ ID NO: 2) resulting from the addition of a sequence comprising 10 amino acid residues (xxx(N/H/E/D)(D/E)xx(N/K)xP) to the C terminus of the amino acid sequence as shown in SEQ ID NO: 1. Specifically, the mutant gene of the present invention results from the substitution of the 12th amino acid residue (threonine (T) or serine (S)) from the N terminus of the consensus sequence resulting from the addition of a given sequence (xxx(N/H/E/D)(D/E)xx(N/K)xP) to the C terminus of the amino acid sequence as shown in SEQ ID NO: 1 with another amino acid, and it encodes a protein having the function of regulating the oil-and-fat amount in a plant.

Genes encoding a protein having the consensus sequence are not limited to genes of given plant species, and such genes can be identified and isolated from a wide variety of plant species. Specifically, genes encoding a protein having the consensus sequence can be identified and isolated from *Arabidopsis thaliana* of *Brassicaceae,* barley (*Hordeum vulgare*) of *Gramineae,* rice (*Oryza sativa*) of *Gramineae, Brassica rapa* of *Brassicaceae,* and *Populus nigra* of *Salicaceae* (*Populus trichocarpa*, which may be referred to as "black cottonwood" or "cottonwood"). Examples of subspecies of *Brassica rapa* that are classified as members of the same species include *Brassica rapa* var *nippo-oleifera*, *Brassica rapa* var *pekinensis*, *Brassica rapa* var *chinensis*, *Brassica rapa* var *rapa*, *Brassica rapa L. var. hakabura*, *Brassica rapa var. nipposinica, Brassica rapa var. perviridis,* and *Brassica rapa* var *chinensis* (pak choi).

Examples of genes encoding a protein having the consensus sequence of *Arabidopsis thaliana* include the At1g54570 gene identified by the GenBank Accession Number: BT005958, the At5g41130 gene identified by the GenBank Accession Number: NM_123478.3, the At5g41120 gene identified by the GenBank Accession Number: AY09638.1, and the At3g26840 gene identified by the GenBank Accession Number: AF360145.1. An example of a gene encoding a protein having the consensus sequence of barley is the FLbaf127k09 gene identified by the GenBank Accession Number: AK251457.1. Examples of genes encoding a protein having the consensus sequence of rice include the Os01g0361500 gene identified by the GenBank Accession Number: NM_001049558.1, the Os01g0362100 gene identified by the GenBank Accession Number: NM_001049562.1, the Os01g0361700 gene identified by the GenBank Accession Number: NM_001049559.1, and the Os09g0509500 gene identified by the GenBank Accession Number: NM_001070165.1. An example of a gene encoding a protein having the consensus sequence of *Brassica rapa* (e.g., *Brassica rapa* var *nippo-oleifera, Brassica rapa* var *pekinensis, Brassica rapa* var *chinensis, Brassica rapa* var *rapa, Brassica rapa L. var. hakabura*, *Brassica rapa var. nipposinica*, *Brassica rapa var. perviridis,* and *Brassica rapa* var *chinensis* (pak choi)) is the KBrH099I08 gene identified by the GenBank Accession Number: AC189616.1. Examples of genes encoding a protein having the consensus sequence of *Populus nigra* include the gene that is present in the region between nucleotides 41811 and 50309, the gene that is present in the region between nucleotides 62687 and 71216, the gene that is present in the region between nucleotides 89072 and 97258, and the gene that is present in the region between nucleotides 75984 and 83278 in the complete nucleotide sequence of the clone POP002-D20, which has been registered under the GenBank Accession Number: AC213081.1.

The nucleotide sequence of the At1g54570 gene and the amino acid sequence of a protein encoded by the At1g54570 gene are shown in SEQ ID NOs: 3 and 4. The amino acid sequence of a protein encoded by the At5g41130 gene is shown in SEQ ID NO: 5. The amino acid sequence of a protein encoded by the At5g41120 gene is shown in SEQ ID NO: 6. The amino acid sequence of a protein encoded by the At3g26840 gene is shown in SEQ ID NO: 7. The amino acid sequence of a protein encoded by the FLbaf127k09 gene is shown in SEQ ID NO: 8. The amino acid sequence of a protein encoded by the Os01g0361500 gene is shown in SEQ ID NO: 9. The amino acid sequence of a protein encoded by the Os01g0362100 gene is shown in SEQ ID NO: 10. The amino acid sequence of a protein encoded by the Os01g0361700 gene is shown in SEQ ID NO: 11. The amino acid sequence of a protein encoded by the Os09g0509500 gene is shown in SEQ ID NO: 12. The amino acid sequence of a protein encoded by the KBrH099I08 gene is shown in SEQ ID NO: 13. The amino acid sequence of a protein encoded by the gene that is present in the region between nucleotides 41811 and 50309 in the complete nucleotide sequence of the clone POP002-D20, which has been registered under the GenBank Accession Number: AC213081.1, is shown in SEQ ID NO: 14. The amino acid sequence of a protein encoded by the gene that is present in the region between nucleotides 62687 and 71216 in the complete nucleotide sequence of the clone POP002-D20 is shown in SEQ ID NO: 15. The amino acid sequence of a protein encoded by the gene that is present in the region between nucleotides 89072 and 97258 in the complete nucleotide sequence of the clone POP002-D20 is shown in SEQ ID NO: 16. The amino acid sequence of a protein encoded by the gene that is present in the region between nucleotides 75984 and 83278 in the complete nucleotide sequence of the clone POP002-D20 is shown in SEQ ID NO: 17.

The results of alignment analysis of the amino acid sequences shown in SEQ ID NOs: 4 to 17 with the use of the CLUSTAL W (1.83) multiple sequence alignment program (available from DDBJ of the National Institute of Genetics, http://clustalw.ddbj.nig.ac.jp/top-j.html) are shown in Fig. 2 (the default scoring matrix BLOSU was used as the amino acid substitution matrix). As shown in Fig. 2, such 14 types of proteins have the above consensus sequence (the underlined region). The mutant gene of the present invention results from substitution of threonine (T) or serine (S) with another amino acid at a position indicated by an arrow in the multiple alignment shown in Fig. 2. Fig. 3 shows a molecular evolution dendrogram obtained from the results of alignment analysis of 14 types of amino acid sequences shown in Fig. 2.

As a method for substituting threonine (T) or serine (S) with another amino acid at a position indicated by an arrow in the 14 types of proteins shown in Fig. 2, a known genetic engineering technique can be adequately employed. In short, the nucleotide sequence of a wild-type gene encoding a protein into which a mutation is to be introduced is identified, and a mutation can be introduced with the use of, for example, a site-directed mutagenesis kit, so as to encode the protein after substitution. For example, the gene into which a mutation has been introduced can be incorporated into an expression vector and recovered in that state in accordance with a conventional technique. A mutation can be introduced into a gene by a known technique, such as the Kunkel or gapped duplex method, or a technique in accordance therewith. For example, a mutation is introduced with the use of mutagenesis kits utilizing site-directed mutagenesis (e.g., Mutant-K or Mutant-G (manufactured by Takara Bio Inc.)) or the LA PCR in vitro Mutagenesis Series Kit (manufactured by Takara Bio Inc.).

The mutant gene of the present invention is not limited to a gene encoding a protein comprising an amino acid sequence derived from any of the amino acid sequences as shown in SEQ ID NOs: 4 to 17 by substitution of the 12th amino acid residue from the N terminus in the aforementioned consensus sequence (i.e., threonine (T) or serine (S)) with another amino acid. The mutant gene of the present invention may comprise an amino acid sequence that results from mutation, such as deletion, substitution, or addition of a plurality of, and preferably 1 or several, amino acids of an amino acid sequence derived from any of the amino acid sequences as shown in SEQ ID NOs: 4 to 17 by substitution of the 12th amino acid residue from the N terminus in the consensus sequence as shown in SEQ ID NO: 1 (i.e., threonine (T) or serine (S)) with another amino acid. For example, 1 to 10, preferably 1 to 8, and more preferably 1 to 5 amino acids may be deleted, added, or substituted in any of the amino acid sequences as shown in SEQ ID NOs: 4 to 17.

Also, a gene encoding a protein having 70% or higher homology to any of the amino acid sequences as shown in SEQ ID NOs: 4 to 17 and having an amino acid sequence resulting from substitution of the 12th amino acid residue from the N terminus in the aforementioned consensus sequence (i.e., threonine (T) or serine (S)) with another amino acid may be used. Such homology is preferably 80% or higher, more preferably 85% or higher, further preferably 90% or higher, and most preferably 95% or higher.

Deletion, addition, or substitution of amino acids can be realized by modifying a gene encoding the above-mentioned protein via a method known in the art. A mutation can be introduced into a gene via known methods, such as the Kunkel or gapped duplex method, or methods in accordance therewith. For example, a mutation is introduced with the use of mutagenesis kits utilizing site-directed mutagenesis (e.g., Mutant-K or Mutant-G (manufactured by Takara Bio Inc.)) or the LA PCR *in vitro* Mutagenesis Series Kit (manufactured by Takara Bio Inc.). A mutation may be introduced into the mutant gene of the present invention via a method involving the use of chemical mutagens typified by EMS (ethyl methane sulfonate), 5-bromouracil, 2-aminopurine, hydroxylamine, N-methyl-N'-nitro-N-nitrosoguanidine, or other carcinogenic compounds, radiation processing typified by the use of x-rays, alpha rays, beta rays, gamma rays, or ion beams, or ultrasonic processing.

Further, the mutant gene of the present invention comprises DNA hybridizing under stringent conditions to DNA comprising a nucleotide sequence complementary to DNA comprising the nucleotide sequence as shown in SEQ ID NO: 3 and encoding a mutant protein having the function of regulating the oil-and-fat content in seeds. Under stringent conditions, so-called specific hybrids are formed, but non-specific hybrids are not formed. For example, hybridization is carried out at 45°C in the presence of 6× SSC (sodium chloride/sodium citrate), followed by washing at 50°C to 65°C in the presence of 0.2 to 1× SSC and 0.1% SDS. Alternatively, hybridization is carried out at 65°C to 70°C in the presence of 1× SSC, followed by washing at 65°C to 70°C in the presence of 0.3× SSC.

When the nucleotide sequence of the mutant gene of the present invention is identified, the mutant gene can be obtained from a variety of plants via chemical synthesis, PCR using the cloned cDNA template, or hybridization using the DNA fragment having the nucleotide sequence of interest as a probe.

The mutant gene of the present invention described above is functionally expressed in a plant of interest by modifying the wild-type gene, so as to substitute the 12th amino acid residue from the N terminus in the aforementioned consensus sequence (i.e., threonine (T) or serine (S)) with another amino acid in the plant genome. Specifically, a plant having the mutant gene of the present invention may be produced via site-directed mutagenesis as described above. Alternatively, such plant may be produced via homologous recombination between the mutant gene prepared in advance and the wild-type gene in the genome. Further, a plant having the mutant gene of the present invention may be produced by deleting the wild-type gene in the plant genome and introducing the mutant gene in an expressible manner. Furthermore, a plant having the mutant gene of the present invention may be produced by introducing the mutant gene, so that it is overexpressed therein without deleting the wild-type gene in the plant genome.

A method of introducing the mutant gene of the present invention into a plant and a conventional method can be adequately adopted. Examples of vectors for introducing and expressing the mutant gene of the present invention in a plant cell that can be preferably used include pBI vectors, pUC vectors, and pTRA vectors. pBI and pTRA vectors can introduce a target gene into a plant with the use of *Agrobacterium.* pBI binary vectors or intermediate vectors are preferably used, and examples thereof include pBI121, pBI101, pBI101.2, and pBI101.3. pUC vectors can directly introduce a gene into a plant, and examples thereof include pUC18, pUC19, and pUC9. Also, plant virus vectors, such as cauliflower mosaic virus (CaMV), bean golden mosaic virus (BGMV), and tobacco mosaic virus (TMV) vectors, can be used.

The mutant gene of the present invention may not be introduced together with DNA having vector functions, but it may be directly introduced into a plant cell by the particle gun method.

The mutant gene of the present invention is preferably included in DNA that is constructed so as to exhibit the functions of such gene. In addition to a promoter, accordingly, an enhancer, a splicing signal, a poly A addition signal, a selection marker, a 5'-UTR sequence, or the like can be ligated to DNA for gene introduction, according to need. Examples of selection markers include the dihydrofolate reductase gene, the ampicillin resistance gene, the neomycin resistance gene, the hygromycin resistance gene, and the bialaphos resistance gene.

As the "promoter," DNA may not be derived from a plant, provided that such DNA can function in a plant cell and induce expression in a given tissue or at a given growth phase of the plant. Specific examples include 35S promoters of cauliflower mosaic virus (CaMV), promoters of nopaline synthase genes (Pnos), maize or rice ubiquitin promoters, maize or rice actin promoters, and tobacco PR protein promoters.

Any sequence may be used as the "terminator," provided that such sequence can terminate the transcription of the gene transcribed by the promoter. Specific examples include terminators of noparine synthase genes (Tnos) and cauliflower mosaic virus poly A terminators.

An "enhancer" is used for improving the expression efficiency of a target gene. For example, an enhancer region including a sequence located upstream in the CaMV35S promoter is preferable.

A transgenic plant can be prepared in accordance with a conventional technique using an expression vector having the mutant gene of the present invention. A transgenic plant can be obtained by introducing such expression vector into a host cell, so that the introduced mutant gene can be expressed therein. If the mutant gene of the present invention is included in a DNA fragment, which is constructed so as to exhibit the functions of the mutant gene, a transgenic plant can be prepared by the particle gun method while lacking DNA having vector functions. A transgenic plant can be prepared in the following manner. Targets of transformation are plant tissues (including the epidermis, the phloem, the parenchyma, the xylem, the vascular bundle, and the plant organs (e.g., leaves, petals, stems, radicals, and seeds)) or plant cells.

Examples of plants having the mutant gene of the present invention include, but are not limited to, dicotyledonous plants and monocotyledonous plants, such as plants of *Brassicaceae, Gramineae, Solanaceae, Leguminosae*, and *Salicaceae* (see below).

Examples of *Brassicaceae* plants include *Arabidopsis thaliana*, oilseed rape (*Brassica rapa* and *Brassica napus*), cabbage (*Brassica oleracea var. capitata*), rapeseed (*Brassica rapa* and *Brassica napus*), field mustard (*Brassica rapa* and *Brassica napus*), *Brassica pekinensis* (*Brassica rapa var. pekinensis*), bok choy (*Brassica rapa var. chinensis*), turnip (*Brassica rapa var. rapa*), *Brassica rapa var. hakabura, Brassica rapa var. lancinifolia, Brassica rapa var. peruviridis*, pak choi (*Brassica rapa var. chinensis*), Japanese radish (*Brassica Raphanus sativus*), and horseradish (*Wasabia japonica*).

Examples of *Solanaceae* plants include tobacco plants (*Nicotiana tabacum*), eggplants (*Solanum melongena*), potatoes (*Solaneum tuberosum*), tomatoes (*Lycopersicon lycopersicum*), *capsicum* (*Capsicum annuum*), and Petunia.

Examples of *Leguminosae* plants include soybeans (*Glycine max*), peas (*Pisum sativum*), horse beans (*Vicia faba*), *Wisteria floribunda,* peanuts (*Arachis. hypogaea*), bird's-foot trefoils (*Lotus corniculatus var. japonicus*), bush beans (*Phaseolus vulgaris*), *azuki* beans (*Vigna angularis*), and Acacia.

Examples of *Compositae* plants include Chrysanthemums (*Chrysanthemum morifolium*) and sunflowers (*Helianthus annuus*).

Examples of *Arecaceae* plants include *Elaeis guineensis* (or *Elaeis oleifera*), *Cocos nucifera, Phoenix dactylifera,* and *Copernicia.*

Examples of *Anacardiaceae* plants include *Rhus succedanea*, *Anacardium occidentale*, *Toxicodendron vernicifluum*, *Mangifera indica*, and pistachio (*Pistacia vera*).

Examples of *Cucurbitaceae* plants include pumpkins (*Cucurbita maxima*, *Cucurbita moschata,* or *Cucurbita pepo*), cucumbers (*Cucumis sativus*), *Trichosanthes cucumeroides,* and gourds (*Lagenaria siceraria var. gourda*).

Examples of *Rosaceae* plants include almonds (*Amygdalus communis*), roses (*Rosa*), strawberries (*Fragaria*), cherry trees (*Prunus*), and apples (*Malus pumila var. domestica*).

Examples of *Caryophyllaceae* plants include carnations (*Dianthus caryophyllus*).

Examples of *Salicaceae* plants include *Populus nigra* (*Populus trichocarpa*, *Populus nigra*, or *Populus tremula*).

Examples of *Gramineae* plants include maize (*Zea mays*), rice (*Oryza sativa*), barley (*Hordeum vulgare*), wheat (*Triticum aestivum*), bamboo (*Phyllostachys*), and sugarcane (*Saccharum officinarum*).

Examples of *Liliaceae* plants include tulips (*Tulipa*) and lilies (*Lilium*).

Examples of methods for introducing an expression vector or a DNA fragment having the mutant gene of the present invention into a plant include the *Agrobacterium* method, the PEG-calcium phosphate method, the electroporation method, the liposome method, the particle gun method (the bombardment method), and the microinjection method. When the *Agrobacterium* method is employed, for example, protoplasts or tissue sections are used. When protoplasts are used, protoplasts may be subjected to coculture with *Agrobacterium* having Ti plasmids or may be fused to *Agrobacterium* spheroplasts (i.e., the spheroplast method). When tissue sections are used, tissue sections may be infected with an aseptically-cultured leaf disc of a target plant via a leaf disk (the leaf disc method), tissue sections may be infected with calluses (undifferentiated cultured cells), or tissue sections may directly infiltrate flower tissue. When transforming monocotyledonous plants by the *Agrobacterium* method, acetosyringone can be used to enhance the efficiency of transformation.

Whether or not the mutant gene has been incorporated into a plant can be confirmed via PCR, Southern hybridization, Northern hybridization, or other techniques. For example, DNA is prepared from a transgenic plant, DNA-specific primers are designed, and PCR is carried out. After PCR is performed, the amplification product may be subjected to agarose gel electrophoresis, polyacrylamide gel electrophoresis, or capillary electrophoresis, the resultant is stained with ethidium bromide, SYBR Green, or the like, and the amplification product is detected as a band. Thus, transformation can be confirmed. Alternatively, PCR may be carried out with the use of primers that have been labeled with fluorescent dyes or the like in advance, and the amplification product can be detected. Further, the amplification product may be bound to a solid phase, such as a microplate, and the amplification product may be detected via fluorescent, enzyme, or other reactions.

As a result of transformation, the resulting tumor tissues, shoots, capillary roots, seeds, or the like can be used for cell culture, tissue culture, or organ culture in that state. Alternatively, conventional plant tissue culture techniques may be employed to reproduce plants therefrom via administration of plant hormones (e.g., auxin, cytokinine, gibberellin, abscisic acid, ethylene, or brassinoride) at adequate concentrations. In general, plants are reproduced from the cultured cell by differentiating the radicals on a medium containing a mixture of adequate types of auxin and cytokinine, transferring the resultants to a medium containing a larger quantity of cytokinine to differentiate shoots, and transferring the resultants to hormone-free soils.

Thus, a transgenic plant into which the mutant gene has been introduced exhibits a characteristic phenotype. That is, the total amount of oil-and-fat in a given tissue of the transgenic plant is increased in comparison with that of a wild-type plant into which the mutant gene has not been introduced; however, the oil-and-fat content (%) in the given tissue is decreased in comparison with that of a wild-type plant. In this case, a given tissue may be, for example, a leaf, cotyledon, radical, radical hair, trichome, flower, seed, fruit, scape, radical tuber, tuber, or bulb. A cotyledon, seed, or fruit is preferable, and a seed is more preferable. The oil-and-fat component in seeds obtained from a plant can be quantitatively detected via a conventional technique. The oil-and-fat component in a given tissue can be measured by a method involving the use of a pulsed NMR apparatus, for example.

The mutant gene of the present invention was identified by visualizing, with fluorescence, oil bodies contained in the cotyledons sampled from the transgenic *Arabidopsis thaliana* plant into which the oleosin-GFP fusion gene had been introduced. In the present invention, however, oil-body-specific proteins are not limited to oleosins, and a wide variety of proteins having oil body specificity can be used. Examples of oil-body-specific proteins include oleosins, steroleosins, and caleosins. In the present invention, any protein may be used as a fluorescent protein that allows visualization of oil bodies in cotyledons, provided that such protein has fluorescence ability. Examples of proteins that can be used include GFP (green fluorescent protein), YFP (yellow fluorescent protein), RFP (red fluorescent protein), OFP (orange fluorescent protein), and BFP (blue fluorescent protein). Such proteins are not limited to fluorescent proteins, as long as the protein allows visualization of oil bodies in cotyledons. A wide variety of proteins that can detect oil bodies with the aid of visible light can be used. A specific example of a protein that can detect oil bodies with the aid of visible light is a luminescent protein, such as luciferase.

Hereafter, the present invention is described in greater detail with reference to the examples, although the technical scope of the present invention is not limited to the following examples.

### [Example 1]

In this example, *Arabidopsis thaliana* plants that are extensively used as model plants were transformed so as to express an oleosin-GFP fusion gene, and transgenic plants in which oil bodies can be observed with the aid of fluorescence were prepared. Thereafter, the resulting transgenic plants were subjected to mutation, and mutants exhibiting varied oil-and-fat amounts in seeds were identified using changes in oil body properties as an indicator. Thereafter, the causal genes in the identified mutants were specified, the genes having the function of varying the oil-and-fat amount in seeds were identified, and a mutation of amino acid substitution that varies the oil-and-fat amount in plants was identified. Hereafter, the concrete process of experiment and the results of experiment are described in detail.

### Material and method

### <Plant material>

The *Arabidopsis thaliana* ecotype *Columbia* was used. In accordance with a conventional technique, plants were subjected to seed sterilization and germination in sterile agar medium (1/2 Murashige and Skoog medium, 0.8% agar) for 7 days at 22°C under light. Thereafter, plants were planted in a pot containing vermiculite and pearlite (1:1) and grown at 22°C for 16 hours under light and for 8 hours in the dark.

### <Preparation of oleosin-GFP gene>

RNA was isolated from the sheath of *Arabidopsis thaliana* using an RNeasy plant mini kit (Quiagen) and subjected to reverse transcription using the SuperScript III first strand synthesis system for RT-PCR (Invitrogen). PCR was carried out using the obtained cDNA, primer 1 (^{5'}AAAAAGCAGGCTCAATGGCGGATACAGCTAGAGGA^{3'}: SEQ ID NO: 18), and primer 2 (⁵'CTCGCCCTTGCTCACCATAGTAGTGTGCTGGCCACC^{3'}: SEQ ID NO: 19), and DNA fragment A having part of the attB1 sequence and part of the GFP gene at both ends of oleosin S3 cDNA was amplified. Separately, PCR was carried out using cDNA encoding the green fluorescent protein (GFP) from Aequorea victoria, primer 3 (^{5'}GGTGGCCAGCACACTACTATGGTGAGCAAGGGCGAG^{3'}: SEQ ID NO: 20), and primer 4 (^{5'}AGAAAGCTGGGTCTTACTTGTACAGCTCGTCCAT^{3'}: SEQ ID NO: 21), and DNA fragment B having part of oleosin S3 cDNA and part of the attB2 sequence added to both ends of GFP cDNA was amplified. Subsequently, DNA fragment A, DNA fragment B, primer 5 (^{5'}GGGG ACA AGT TTG TAC AAA AAA GCA GGC T^{3'}: SEQ ID NO: 22), and primer 6 (^{5'}GGGG AC CAC TTT GTA CAA GAA AGC TGG G^{3'}: SEQ ID NO: 23) were mixed, and PCR was further carried out to prepare an oleosin-GFP fusion gene having the attB1 sequence and the attB2 sequence at both ends. The nucleotide sequence of the oleosin-GFP fusion gene and the amino acid sequence of the gene product are shown in SEQ ID NOs: 24 and 25.

The obtained fusion gene was cloned into a Ti vector having the attR1 and attR2 sequences downstream of the CaMV 35S promoter and having a kanamycin-resistant marker with the aid of the pDONR221 vector in accordance with the protocol of the Gateway system (Invitrogen). The obtained plasmid was introduced into *Agrobacterium* (*Agrobacterium tumefacience* C58C1 rifR) via electroporation, and the resultant was designated as Ti-OleG.

### <Transformation into Arabidopsis thaliana>

The oleosin-GFP fusion gene was introduced into the genome of *Arabidopsis thaliana* by the *Agrobacterium* method. At the outset, Ti-OleG was allowed to grow in YEB medium (5 g/l polypeptone, 5 g/l beef extract, 1 g/l yeast extract, 5 g/l sucrose, 0.5 g/l MgSO₄) until the absorbance at 600 nm (A600) reached 0.8 to 1.0 at 28°C, and it was then harvested via centrifugation. The resulting cells were suspended in an infiltration solution (10 mM MgCl₂, 5% sucrose, 0.05% Silwet L-77) so as to adjust A600 at 0.8. After scapes of flowering *Arabidopsis thaliana* were soaked in the suspension for 1 minute, fructified seeds were collected. The collected seeds were sterilized and then sowed in sterile agar medium containing 25 mgm/l kanamycin, and the transgenic *Arabidopsis thaliana* plant into the genome of which the oleosin-GFP fusion gene had been inserted was isolated using kanamycin resistance as an indicator. Seeds were obtained from the resulting transgenic *Arabidopsis thaliana* plant, and progenies of the transgenic plants, which were homozygous for the kanamycin resistance marker, were selected and designated as OleG.

### <Transgenic plant mutagen treatment>

OleG seeds were treated with a 0.2% ethyl methane sulfonate solution for 16 hours and then sowed in a pot containing vermiculite and pearlite (1:1). The seeds were grown at 22°C under light for 16 hours and in the dark for 8 hours, and the progeny seeds were collected and designated as M2 seeds.

### <GFP fluorescence observation>

Mutants were screened for using a fluorescence stereomicroscope (Carl Zeiss SteREO Lumar V12). The OleG and M2 seeds were vertically positioned on sterile agar medium and germinated in the dark for 6 days. The GFP fluorescence of the oleosin-GFP fusion protein in cells of etiolated cotyledons, embryonic axes, and radicals was observed under a fluorescence stereomicroscope (Carl Zeiss). Seeds having GFP fluorescence intensities and distributions different from those of OleG were identified as mutants.

In order to compare GFP fluorescence of the oleosin-GFP fusion protein of the OleG and mutant seeds, a confocal laser microscope (Carl Zeiss LSM 510) was used. Etiolated cotyledons, embryonic axes, and radicals were cut from the OleG and mutant seeds, which had been germinated in the dark for 6 days, and they were then mounted on a glass slide. GFP fluorescent images of the cells were photographed under the same conditions, the frequency distribution of fluorescence intensity relative to pixels within the same area was calculated using image analyzing software included with the microscope, and the fluorescence sum (i.e., a = sum; fluorescence intensity x pixel number) was determined.

### <Electrophoresis and immunoblot analysis of seed protein>

20 seed grains were pulverized in 40 µl of SDS sample buffer, and the supernatant resulting from centrifugation was designated as a seed protein sample. In accordance with a conventional technique, 15 µl of the sample was subjected to SDS polyacrylamide electrophoresis. The electrophoresed gel was stained with a solution of 0.2% Coomassie brilliant blue R-250 (containing 25% methanol and 10% acetic acid).

Immunoblot analysis was carried out by subjecting 5 µl of the sample to SDS polyacrylamide gel electrophoresis and transferring the proteins in the gel onto a nitrocellulose membrane via semi-dry blotting. Detection of the proteins that had been transferred onto the nitrocellulose membrane with the use of an anti-protein antibody was carried out in accordance with the protocols of GE Healthcare Bio-Sciences using ECL Western blotting detection reagents. In this case, 1/5000-fold diluted antibodies were used as the primary antibodies (the anti-oleosin antibodies or the anti-GFP antibodies) and the secondary antibodies. Luminescence was detected using a luminescent image analyzer (LAS-1000 plus, Fujifilm Corporation).

### <Electron microscopic seed cell observation>

Half seeds were immobilized using a fixative (4% paraformaldehyde, 1% glutaraldehyde, 10% DMSO, 0.05M cacodylate buffer (pH 7.4)). The immobilized sample was embedded in epon 812 resin and ultrathin sections were prepared using a Leica Microtome Ultracut UCT. The ultrathin sections were subjected to electron staining with 4% uranium acetate and 0.4% lead citrate and then observed under an electron microscope (H-7600, Hitachi, Ltd.).

### <Measurement of oil-and-fat amount in seed>

While performing neutralization of static electricity, the seeds were weighed with an accurate electron balance using drug packing paper, and 10 to 12 mg of seeds were fractionated. The seeds were introduced into a test tube for pulsed NMR, and the oil-and-fat content in seeds (% by weight) was determined using a MARAN-23 pulsed NMR (Resonance Instruments) based on the ¹H-pulsed NMR relaxation times. The detailed measurement procedure described in the instructions for pulsed-NMR measurements was employed.

### <Analysis of fatty acid composition in seed oil-and-fat>

About 1 mg to 5 mg of seed samples were fractionated and introduced into a 1.5-ml micro test tube. A tungsten carbide bead (φ = 3 mm) was added to the micro test tube, and 450 µl of methanol, 50 µl of a solution of butyl hydroxyl toluene mixed with a methanol solvent at 0.2% (w/v), and 10 µl of 0.2% C15:0 fatty acid as an internal standard material were further added. The micro test tube to which such various reagents and the sample had been added was subjected to oscillation at a frequency of 1/20 s using a Retsch Mixer Mill Type MM301 for 1 minute, and the seeds were pulverized. The sample was transferred to a 10-ml test tube with a screw cap. Further, the inside of the micro test tube was washed twice with 250 µl of methanol, and the methanol wash solution was added to the test tube to adjust the amount of the sample solution to about 1 ml. A 10% hydrochloric acid/methanol solution (1 ml) was added thereto, the mixture was treated at 80°C for 1 hour, 1.5 ml of n-hexane was added thereto, the mixture was agitated using a vortex mixer, and the n-hexane layer was transferred to a 10-ml spit tube. Further, the inside of the test tube used for methanolysis was washed with 1 ml of n-hexane, and the n-hexane-washed layer was added to the spit tube. The resulting n-hexane solvent solution was subjected to nitrogen gas purging at 40°C, and fatty acid methyl ester was dried. The dried fatty acid methyl ester was dissolved in 500 µl of n-hexane, and various fatty acid methyl esters were separated and quantified via GC-FID. Quantification was carried out with reference to the area of the internal standard (C15:0 fatty acid).

### <Identification of mutant gene>

After the mutagen treatment, mutants having a *Columbia* ecotype background were crossed with wild-type *Arabidopsis thaliana* plants (i.e., the ecotype *Landsberg erecta*) in order to perform high-precision mapping of causal genes for mutants exhibiting changed oil-and-fat amounts in seeds, and 310 lineages of the F2 generation that were homozygous for the mutant gene were obtained. In accordance with a conventional technique, the genetic backgrounds of these 310 lineages of chromosomes (620 chromosomes in total) were scored using many molecular markers. The gene that was deduced to be the causal gene based on the genome DNAs of the OleG and mutant seeds was amplified via PCR, and the nucleotide sequence thereof was determined using a 3130xl Genetic Analyzer (Applied Biosystems).

### Results and Discussion

### <Establishment of method for screening for mutant that is deficient in oil body formation>

The fusion gene (oleosin-GFP) encoding the fusion protein of oleosin and green fluorescent protein (GFP) was prepared and then ligated to a site downstream of DNA of the 35S promoter of the cauliflower mosaic virus (Fig. 4A). The DNA construct was introduced into genome DNA of *Arabidopsis thaliana* by the *Agrobacterium* method to prepare a transgenic *Arabidopsis thaliana* plant, and the resultant was designated as oleG. The results of observation of oleG cotyledons, which had been germinated in the dark for 6 days, using a fluorescent microscope are shown in Fig. 4B. As is apparent from Fig. 4B, an oil body membrane is labeled with GFP fluorescence, and aggregates of many small oil bodies are present. Further, the presence of oil bodies in germs and green cotyledons, true leaves, and petals, which had been germinated under light, was observed, in addition to cotyledons, which had been germinated in the dark.

In order to identify the gene associated with the mechanism for accumulating plant oil-and-fats in oil bodies, the oleG seeds were subjected to mutation with ethyl methane sulfonate to obtain progeny M2 seeds. The M2 plants, which had been germinated in the dark for 6 days, were observed under a fluorescent microscope, and mutant A (Fig. 4C) and mutant B (Fig. 4D) exhibiting fluorescent intensity different from that of oleG were obtained. The GFP fluorescent intensity in the germinated cotyledons of such mutants was lower than that of oleG.

### <Correlation between sum of GFP fluorescence and oil-and-fat content in seed>

Confocal laser microscope photographs of GFP fluorescence of cotyledons of OleG, mutant A, and mutant B, which had been germinated in the dark for 6 days, under the same conditions in the same area with the same pixel number were obtained, and the sum of GFP fluorescence (i.e., a = sum; fluorescence intensity x pixel number) was determined based on the frequency distribution of the pixel number relative to the GFP fluorescence intensity of each photograph. When the sum of OleG fluorescence was designated as 100%, the fluorescence sum of mutant A was 37.9%, and that of mutant B was 85.1%. Separately, the oil-and-fat contents in seeds of OleG, mutant A, and mutant B were measured and found to be 34.66% ± 0.43%, 26.91% ± 0.34%, and 32.34% ± 0.49%, respectively. A positive correlation was observed between the sum of GFP fluorescence and the oil-and-fat content in seeds (Fig. 5). Also, the fatty acid compositions in lipids accumulated in seeds of such mutants were analyzed (Fig. 6). As a result, the C20:1 (11)cis content in mutant A and mutant B were found to be lower than that in OleG. In this description, notation regarding a fatty acid is provided in the following manner; C(the number of carbon atoms:"the number of unsaturated carbon bonds""(the carbon number counted from the carboxyl group side where an unsaturated bond exists)""the cis/trans geometric isomerization of unsaturated bonds". When a cis/trans geometric isomerism is not known, notation regarding such isomerism is not provided. For example, stearic acid is represented as C18:0 and general oleic acid is represented as C:18:1(9)cis. In mutant A, an increase was observed in C18:3 (9, 12, 15), and an increase was observed in C18:1(9)cis in mutant B. Thus, measurement of the sum of GFP fluorescence of the oleosin-GFP fusion protein was found to enable identification of a mutant having an abnormality in the mechanism for accumulating plant oil-and-fats alive.

In Fig. 6, bar charts representing fatty acids represent OleG, mutant A, and mutant B in that order from the left.

### <Analysis of seed protein>

Fig. 7 shows the results of analysis of proteins contained in seeds of a wild-type *Arabidopsis thaliana* plant, OleG, mutant A, and mutant B. The results of Coomassie brilliant blue staining (Fig. 7A), immunoblot analysis using the anti-oleosin antibody (Fig. 7B), and immunoblot analysis using the anti-GFP antibody (Fig. 7C) demonstrate that the amounts of endogenous oleosins and of oleosin-GFP decreased in seeds of mutant A and mutant B, in comparison with those of OleG. In Figs. 7A to 7C, lane 1 represents a sample derived from a wild-type *Arabidopsis thaliana* plant, lane 2 represents a sample derived from OleG, lane 3 represents a sample derived from mutant A, and lane 4 represents a sample derived from mutant B.

### <Electron microscopic observation of oil bodies>

Fig. 8 shows the results of observation of OleG, mutant A, and mutant B seed cells under an electron miscroscope. OleG seed cells are filled with small oil bodies, as in the case of wild-type *Arabidopsis thaliana* plants. In contrast, gaps that are deduced to be cytosols are observed between oil bodies, and each oil body is round in mutant A and mutant B. This is considered to result from a decreased amount of seed reserved lipid. Also, several oil bodies in seeds are gigantic. This is considered to result from a decreased amount of oleosin in the oil body membrane.

### <Identification of causal gene>

Mutant A having a *Columbia* ecotype background was crossed with an wild-type *Arabidopsis thaliana* plant having a *Landsberg erecta* ecotype background, and high-precision mapping of causal genes was carried out. The results of scoring of the genetic backgrounds of 620 chromosomes with the use of molecular markers are shown in Fig. 9A. The number of *Landsberg erecta*-derived chromosomes indicates that recombination took place between the locus of the causal gene of mutant A and a molecular marker. As shown in Fig. 9A, high-precision mapping demonstrates that the locus of the causal gene of mutant A is located in a position very close to the first chromosome At1g54560, which is between At1g54150 and At1g54970. The presence of 87 genes including the At1g54570 gene, whose functions are unidentified (Fig. 9B), is suggested in this region.

At1g54570 encodes a polypeptide including a portion that is proposed as the diacylglycerol acyltransferase (DAGAT) domain. The DAGAT domain is an amino acid sequence that is considered to be commonly present in an enzyme associated with triacylglycerol synthesis (i.e., diacylglycerol acyltransferase). Since this is a deduction based on nucleotide sequence information, polypeptides having the DAGAT domain do not always have DAGAT activity. Also, research regarding DAGAT has been conducted mainly with the use of budding yeast, and no case is known in which a given polypeptide in plant seeds has been identified as DAGAT.

The nucleotide sequence of the At1g54570 gene region located in genome DNA of mutant A was analyzed. As a result, a point mutation of C3252T was observed in the nucleotide sequence of At1g54570 (the notation "C3252T" indicates substitution of C with T at nucleotide 3252, when A of the initiation codon ^{5'}ATG^{3'} of cDNA relative to mRNA is designated as 1). This point mutation is present in exon 10, and it induces missense mutation of T508I in the DAGAT domain of the amino acid sequence of the At1g54570 gene product (the notation "T508I" indicates a substitution of T(Thr) at the 508th amino acid residue from the N-terminus of a polypeptide with I(Ile)). Thus, it was concluded that the causal gene of mutant A is At1g54570, and the mutation of T508I in the At1g54570 gene product is an amino acid substitution that varies the oil-and-fat amount in a plant.

### Industrial Applicability

The mutant gene of the present invention can regulate the oil-and-fat content or oil-and-fat composition in seeds. According to the method for regulating the oil-and-fat content in seeds of the present invention, a plant with the regulated oil-and-fat content or oil-and-fat composition can be provided.

All publications, patents, and patent applications cited herein are incorporated herein by reference in their entirety.

## Claims

1. A mutant gene encoding a mutant protein having a consensus sequence derived from the amino acid sequence as shown in SEQ ID NO: 1 by substitution of the 12th amino acid residue from the N terminus with another amino acid and having the function of regulating the oil-and-fat content in plant seeds.

2. The mutant gene according to claim 1, wherein the other amino acid is an amino acid selected from the group consisting of isoleucine, valine, leucine, and methionine.

3. The mutant gene according to claim 1, wherein the other amino acid is isoleucine.

4. The mutant gene according to claim 1, wherein the mutant protein has an amino acid sequence derived from an amino acid sequence as shown in any one of SEQ ID NOs: 4 to 17 comprising a substitution of the 12th amino acid residue from the N terminus in the consensus sequence with another amino acid.

5. The mutant gene according to claim 1, which is a mutant of a plant-derived gene.

6. The mutant gene according to claim 1, which is a mutant of a gene derived from a plant belonging to a family selected from the group consisting of *Gramineae, Brassicaceae,* and *Salicaceae.*

7. The mutant gene according to claim 1, which is a mutant of a gene derived from a plant belonging to a species selected from the group consisting of barley, rice, *Arabidopsis thaliana, Brassica rapa,* and *Populus nigra.*

8. A mutant protein encoded by the mutant gene according to any one of claims 1 to 7.

9. A transgenic cell into which the mutant gene according to any one of claims 1 to 7 is introduced.

10. A transgenic plant into which the mutant gene according to any one of claims 1 to 7 is introduced.

11. A method for regulating the oil-and-fat content in seeds comprising introducing a mutation into a gene encoding a protein having a consensus sequence comprising the amino acid sequence as shown in SEQ ID NO: 1, wherein the mutation id a substitution of the 12th amino acid residue from the N terminus of the amino acid sequence as shown in SEQ ID NO: 1 with another amino acid.

12. The method for regulating the oil-and-fat content in seeds according to claim 11, wherein the other amino acid is an amino acid selected from the group consisting of isoleucine, valine, leucine, and methionine.

13. The method for regulating the oil-and-fat content in seeds according to claim 11, wherein the other amino acid is isoleucine.

14. The method for regulating the oil-and-fat content in seeds according to claim 11, wherein a mutant protein resulting from substitution of the 12th amino acid residue from the N terminus with another amino acid in the consensus sequence included in an amino acid sequence as shown in any one of SEQ ID NOs: 4 to 17 is expressed.

15. The method for regulating the oil-and-fat content in seeds according to claim 11, wherein a gene derived from a plant belonging to a family selected from the group consisting of *Gramineae, Brassicaceae,* and *Salicaceae* is varied.

16. The method for regulating the oil-and-fat content in seeds according to claim 11, wherein a gene derived from a plant belonging to a species selected from the group consisting of barley, rice, *Arabidopsis thaliana, Brassica rapa,* and *Populus nigra* is varied.
